# EUROPEAN PATENT APPLICATION

(11) **EP 4 483 876 A1**
(43) Date of publication of application: **01.01.2025**
(21) Application number: 23759348.8
(22) Date of filing: 27.03.2023
(51) Int. Cl.: A61K 31/4439, A61K 31/444, A61P 19/02, A61P 17/06, C07D 401/12, C07D 401/14, C07D 413/14

(54) **USE OF INDAZOLE COMPOUND FOR TREATING PSORIASIS**

(30) Priority: 25.02.2022 CN 202210179789
(71) Applicant: Wuhan Createrna Science and Technology Co.,Ltd., Wuhan, Hubei 430075 (CN)
(72) Inventor: LUAN, Linbo, Shanghai 200120 (CN); TANG, Chunlan, Shanghai 200120 (CN); CHEN, Yongkai, Shanghai 200120 (CN); WANG, Chaodong, Shanghai 200120 (CN)
(74) Representative: Jones Day
(86) International application number: PCT/CN2023/084168
(87) International publication number: WO 2023/160727

(57) **Abstract**

Provided herein is the use of indazole compounds represented by formula (I), or a stereoisomer, a racemate, a tautomer, an isotopically labeled compound, a prodrug, or a pharmaceutically acceptable salt thereof, in the manufacture of medicament for treating and/or preventing psoriasis. The compound of formula (I) is as follows:

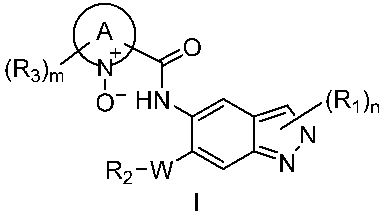

## Description

The present application claims priority to Chinese Patent Application No. 202210179789.6 filed on February 25, 2022 entitled "Use of Indazole Compound for Treating Psoriasis", the entirety of which is incorporated herein by reference.

### TECHNICAL FIELD

The present application relates to the field of medicine, and specifically relates to the use of certain indazole compounds for treating psoriasis.

### BACKGROUND

Psoriasis is an immune-mediated chronic inflammatory disease caused by various factors, including environmental and genetic factors. The etiology of psoriasis is complex, potentially involving T cell differentiation, infiltration of inflammatory cells, and proliferation of keratinocytes. Additionally, heredity, infections, metabolic disorders, endocrine abnormalities, and other related factors (such as smoking, alcohol consumption, trauma, fatigue, and psychological factors) can also induce or even exacerbate psoriasis.

Currently commonly used topical medications for psoriasis include glucocorticoids, retinoic acid, vitamin D derivatives, dithranol, tar preparations, and calcineurin inhibitors. However, the use of dithranol, tar preparations, and glucocorticoids is limited due to their significant side effects, while the therapeutic effects of retinoic acid, calcineurin inhibitors, and vitamin D derivatives are not very satisfactory. Some biologics provide rapid efficacy but are expensive. Traditional Chinese medicines, due to their complex ingredients, often have unclear efficacy and are prone to side effects. Therefore, it is necessary to explore new drugs with good efficacy and safety for the treatment of psoriasis.

### SUMMARY

To address existing technical problems, the present application provides the use of a compound in the manufacture of a medicament for treating and/or preventing psoriasis, wherein the compound is a compound of formula (I): 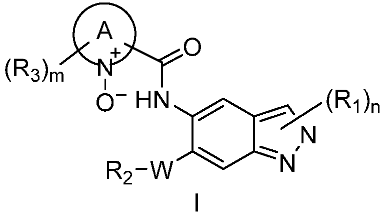 or a stereoisomer, a racemate, a tautomer, an isotopically labeled compound, a prodrug, or a pharmaceutically acceptable salt thereof, wherein:
ring Ais 5- to 14-membered heteroaryl or 5- to 12-membered heterocyclyl comprising at least one N;
R₁, R₂, and R₃ are each independently selected from hydrogen, halogen, CN, OH, or the following groups: (C₁-C₁₂) aliphatic hydrocarbyl, (C₁-C₁₂) aliphatic hydrocarbyl optionally containing one, two, or more heteroatoms, C₃-₁₂ cycloalkyl, 3- to 12-membered heterocyclyl, C₆₋₂₀ aryl, 5- to 14-membered heteroaryl, or -NRₐR_{b}; each of which is optionally substituted by one, two, or more R;
W is selected from O, S, NH, or a single bond;
Rₐ and R_{b} are each independently selected from H or (C₁-C₁₂) aliphatic hydrocarbyl;
each R is independently selected from halogen, CN, OH, SH, NRₐR_{b}, or the following groups: (C₁-C₁₂) aliphatic hydrocarbyl, (C₁-C₁₂) aliphatic hydrocarbyl optionally containing one, two, or more heteroatoms, C₃-₁₂ cycloalkyl, 3- to 12-membered heterocyclyl, C₆₋₂₀ aryl, or 5- to 14-membered heteroaryl; each of which is optionally substituted with one, two, or more R';
each R' is independently selected from halogen, CN, OH, SH, or NRₐR_{b};
n is selected from 1, 2, or 3; m is selected from 1, 2, 3, 4, 5, or 6;
the "(C₁-C₁₂) aliphatic hydrocarbyl optionally containing one, two, or more heteroatoms" is optionally selected from (C₁-C₁₂) aliphatic hydrocarbyloxy, (C₁-C₁₂) aliphatic hydrocarbylthio, (C₁-C₆) aliphatic hydrocarbyloxy-(C₁-C₆) aliphatic hydrocarbyl, (C₁-C₆) aliphatic hydrocarbylthio-(C₁-C₆) aliphatic hydrocarbyl, N-(C₁-C₃) aliphatic hydrocarbylamino-(C₁-C₆) aliphatic hydrocarbyl, or N, N-di-(C₁-C₃) aliphatic hydrocarbylamino-(C₁-C₆) aliphatic hydrocarbyl;
the "5- to 14-membered heteroaryl or 5- to 12-membered heterocyclyl comprising at least one N" is optionally a heteroaryl or heterocyclyl comprising at least one nitrogen atom on the ring, and optionally further comprising additional heteroatoms selected from N, O, or S, such as selected from pyridine, pyrrole, piperidine, or tetrahydropyrrole.
the (C₁-C₁₂) aliphatic hydrocarbyl is optionally selected from (C₁-C₁₂) alkyl, (C₂-C₁₂) alkenyl, or (C₂-C₁₂) alkynyl; preferably, each (C₁-C₁₂) aliphatic hydrocarbyl is selected from (C₁-C₆) alkyl, (C₂-C₆) alkenyl, or (C₂-C₆) alkyny;
the "halogen" is optionally selected from F, Cl, Br, or I;
the "C₃₋₁₂ cycloalkyl" is optionally selected from cyclopropyl, cyclobutyl, cyclopentyl, or cyclohexyl.

In one preferred embodiment, R₁, R₂, and R₃ are each independently selected from the following groups: methyl, ethyl, n-propyl, isopropyl, n-butyl, isobutyl, tert-butyl, n-pentyl, isopentyl, neopentyl, n-hexyl, vinyl, 1-propenyl, 2-propenyl, 1-methylvinyl, 1-butenyl, 1-ethylvinyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl, cyclohexyl, methoxy, ethoxy, propoxy, butoxy, pentyloxy, methoxymethyl, ethoxymethyl, propoxymethyl, methoxyethyl, ethoxyethyl, propoxyethyl, methoxypropyl, ethoxypropyl, propoxypropyl, N-methylaminomethyl, N-methylaminoethyl, N-ethylaminoethyl, N,N-dimethylaminomethyl, N,N-dimethylaminoethyl, N,N-diethylaminoethyl, amino, N,N-dimethylamino, N,N-diethylamino, tetrahydropyrrolyl, piperidyl, pyridyl, pyrazinyl, pyrrolyl, imidazolyl, pyrazolyl, oxazolyl, isoxazolyl, 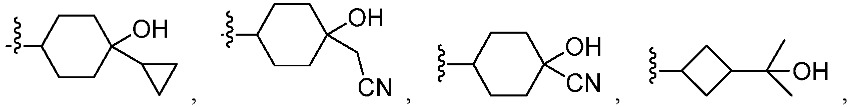 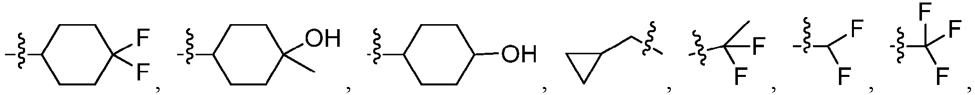 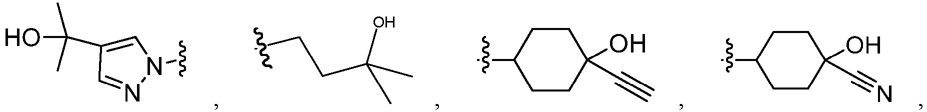 or 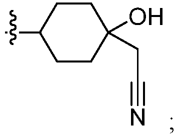 each of which is independently optionally substituted by one, two, or more R. the 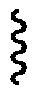 refers to the attachment site of the group.

In one preferred embodiment, the compound of formula I is a compound of formula Ia, formula Ib, formula Ic, formula Id, or formula Ie: 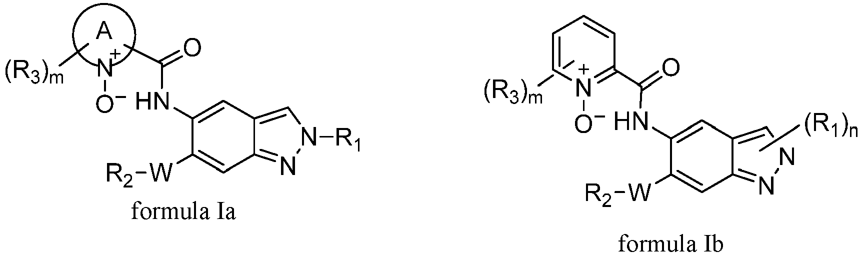 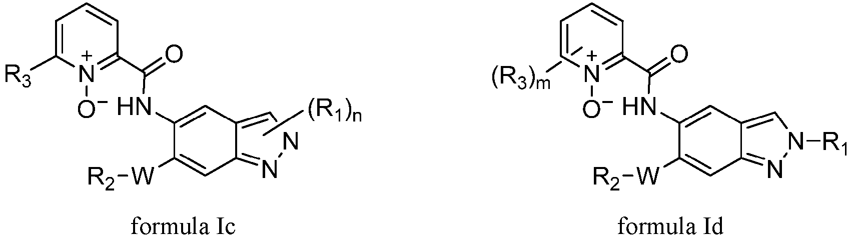 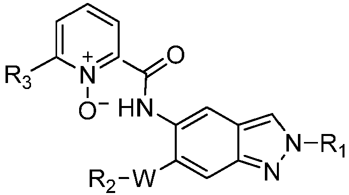 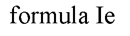 wherein in formula Ia, formula Ib, formula Ic, formula Id, and formula Ie, ring A, R₁, R₂, R₃, m, n, and W are as defined in formula I.

In one more preferred embodiment, the compound of formula I is selected from the following structures:

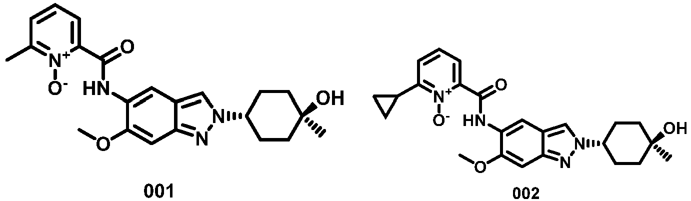

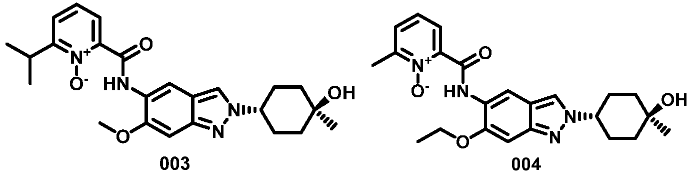

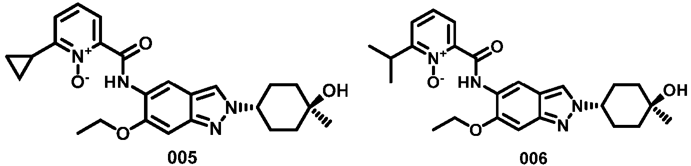

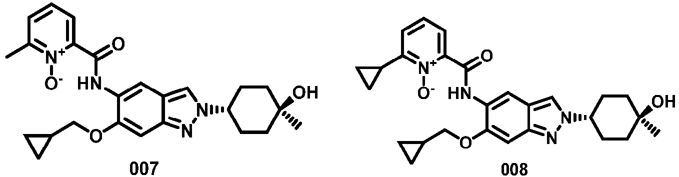

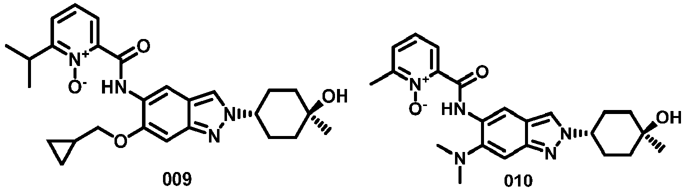

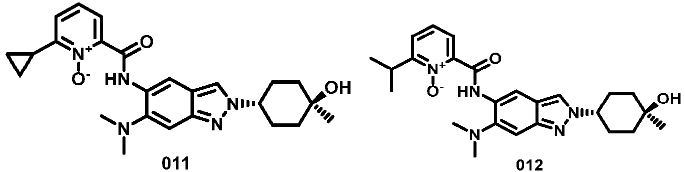

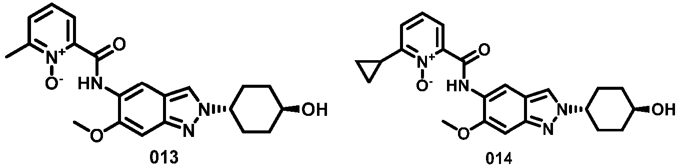

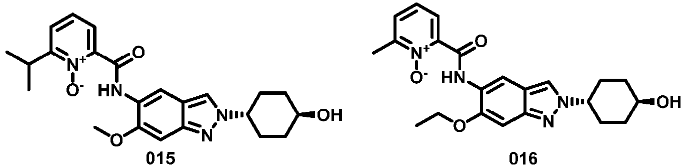

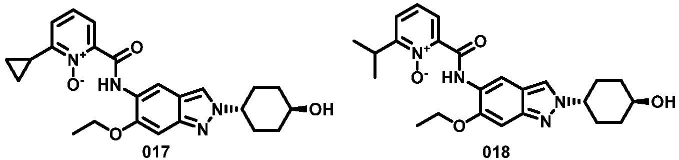

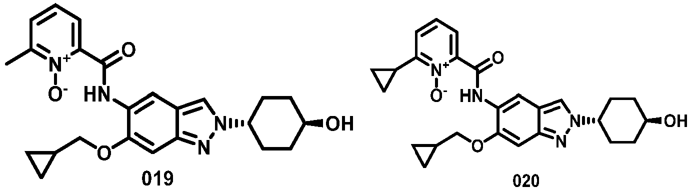

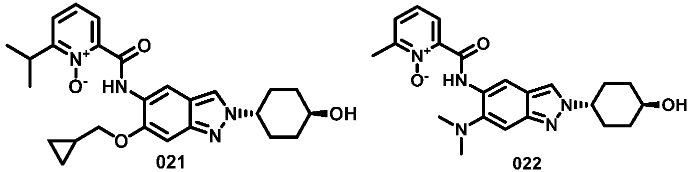

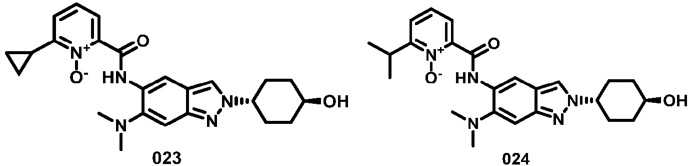

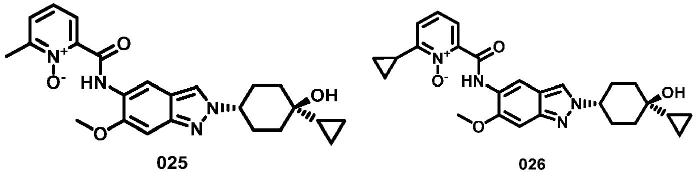

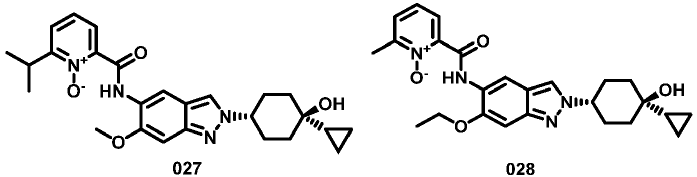

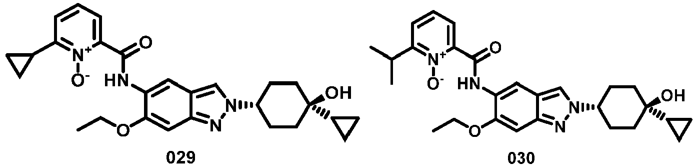

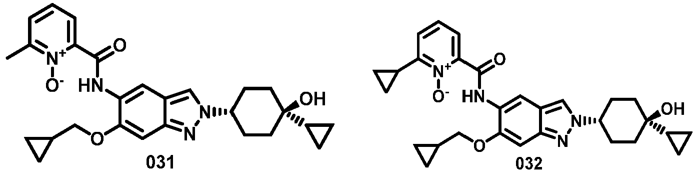

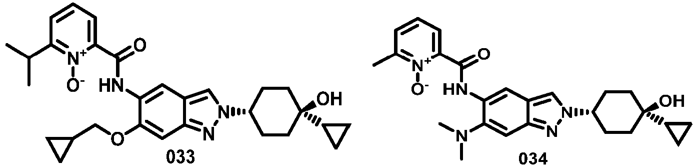

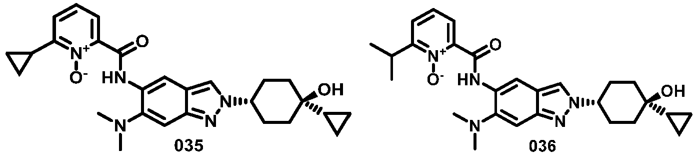

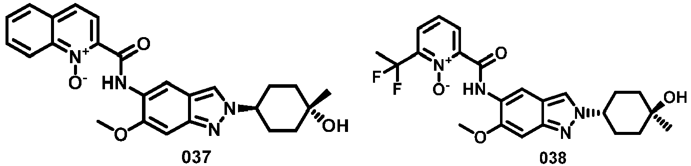

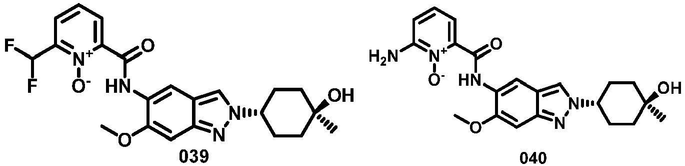

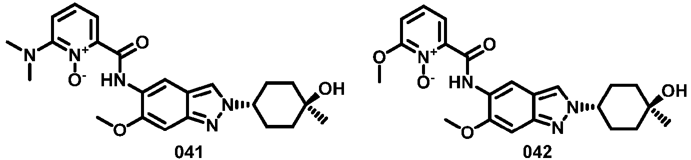

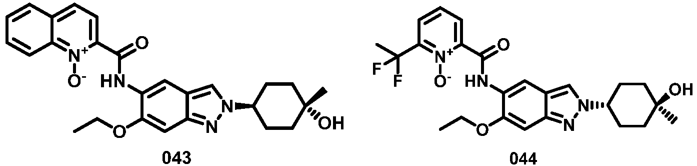

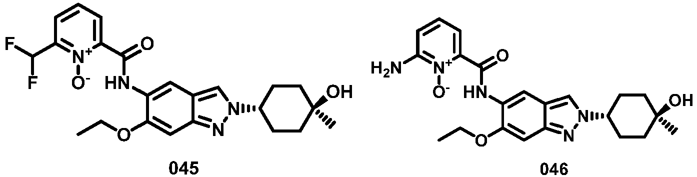

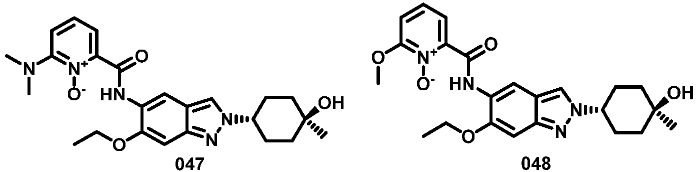

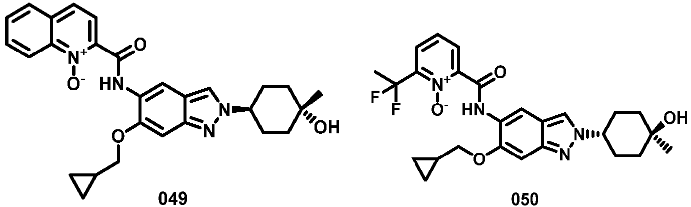

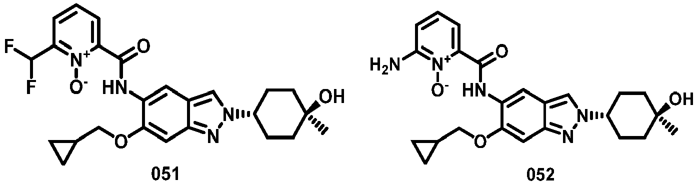

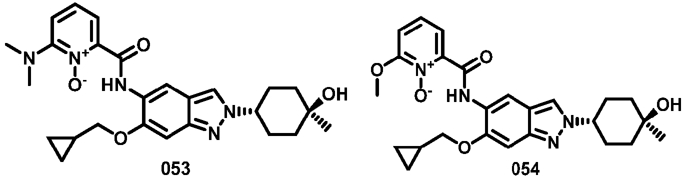

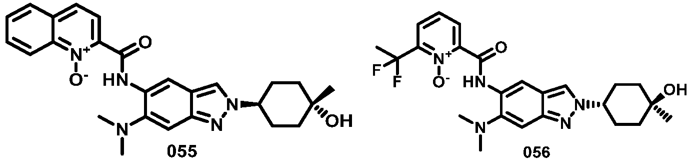

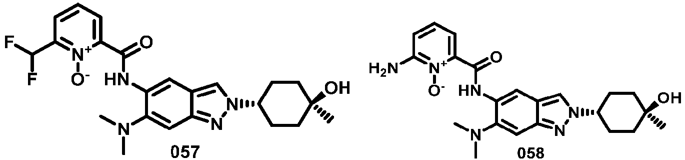

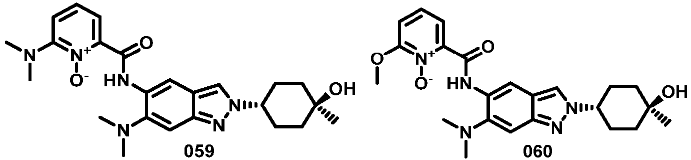

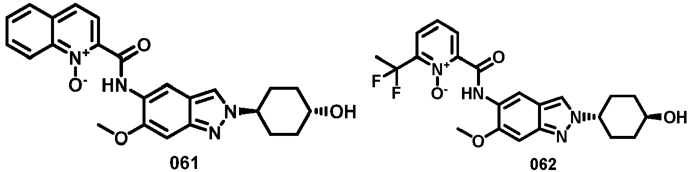

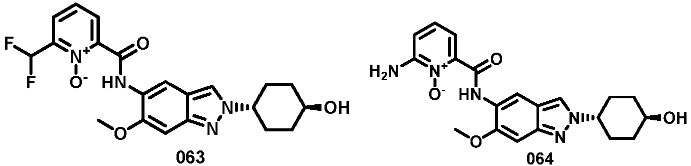

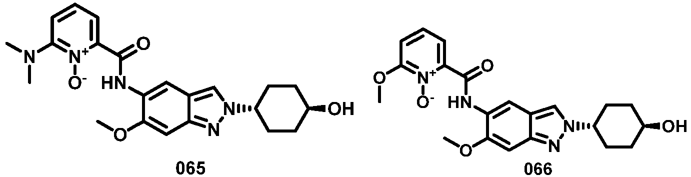

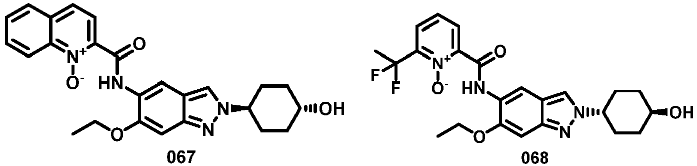

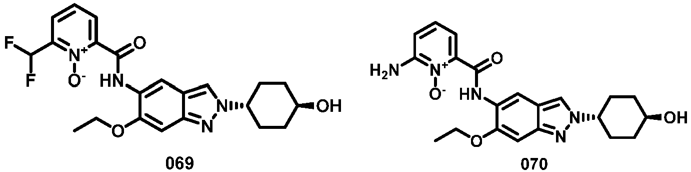

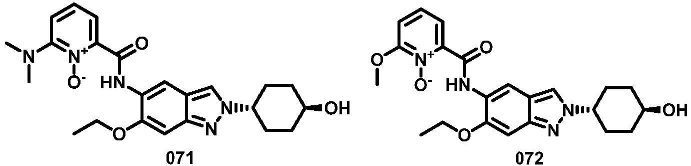

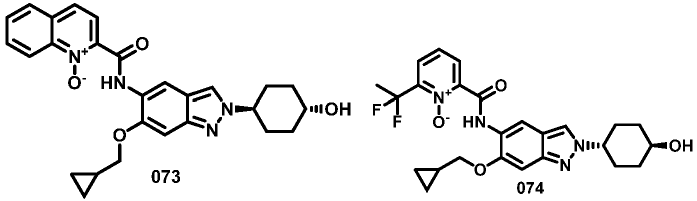

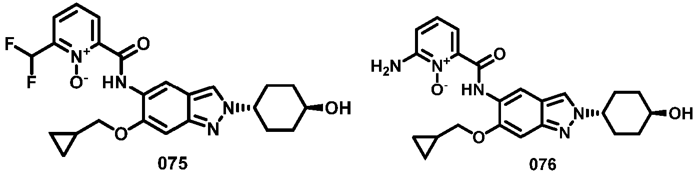

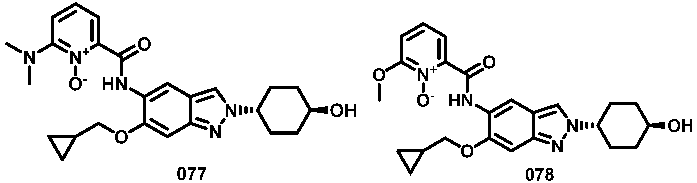

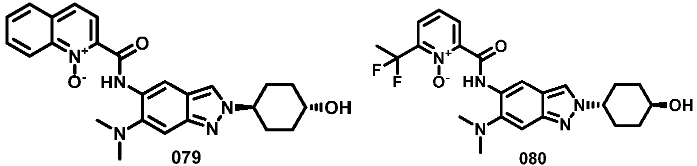

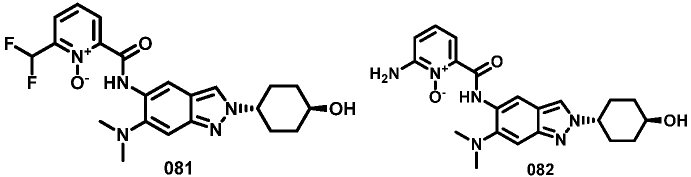

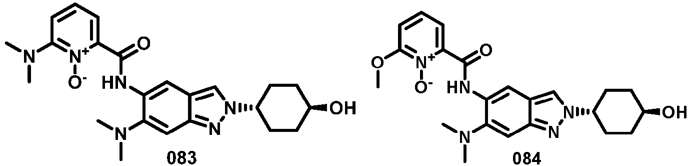

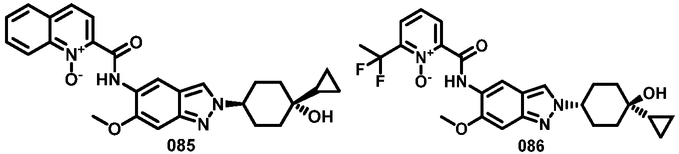

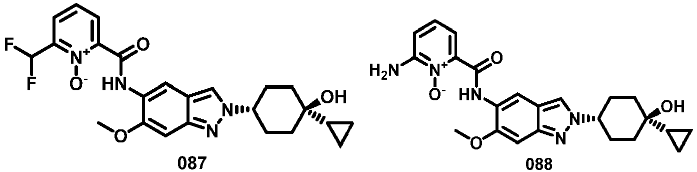

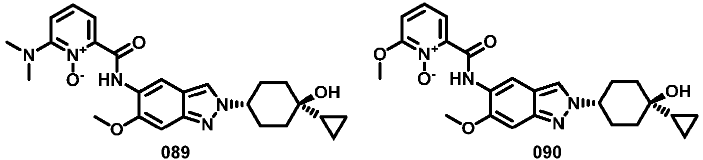

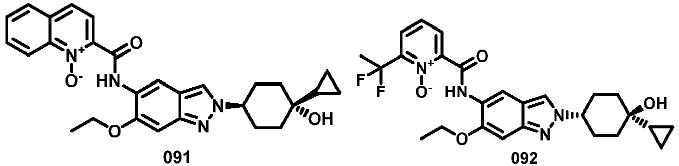

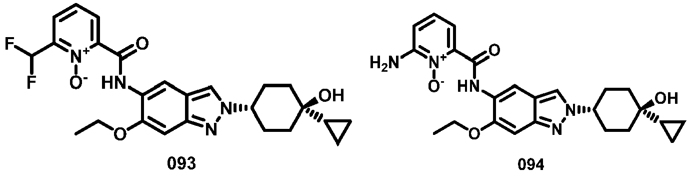

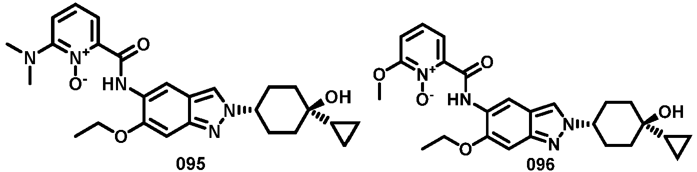

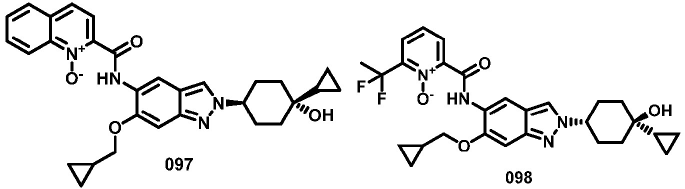

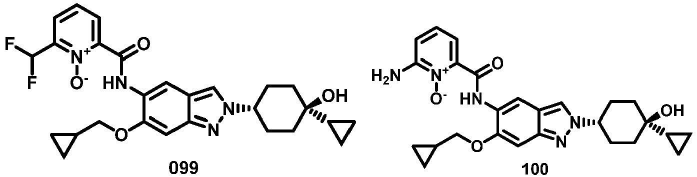

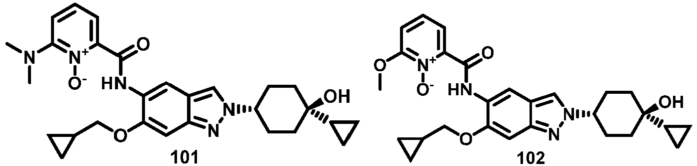

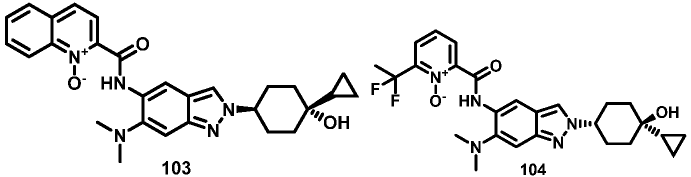

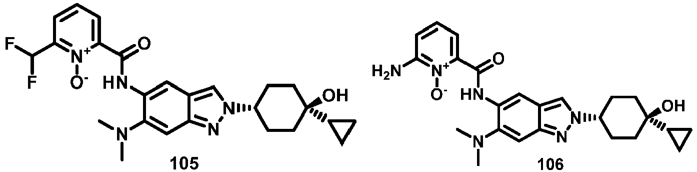

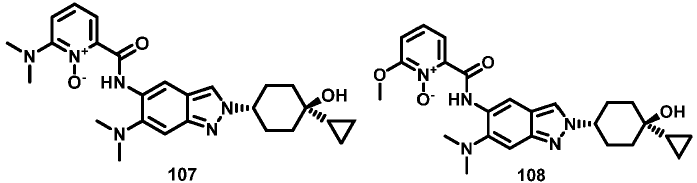

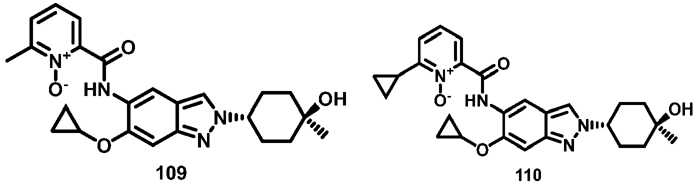

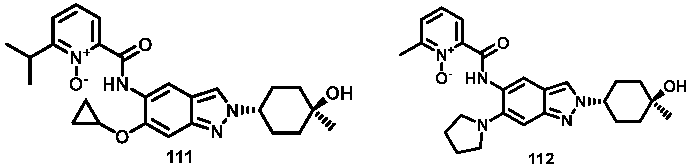

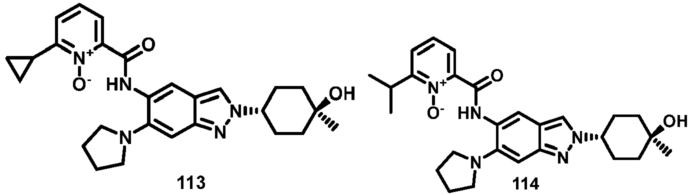

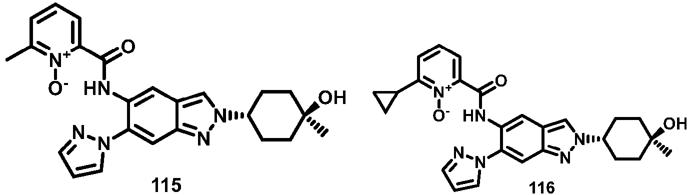

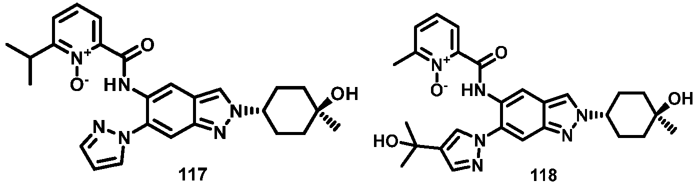

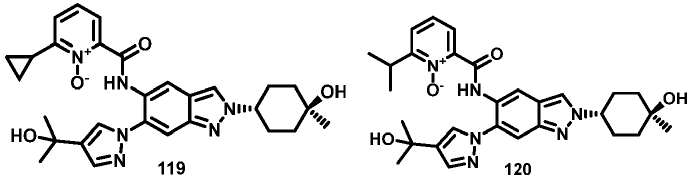

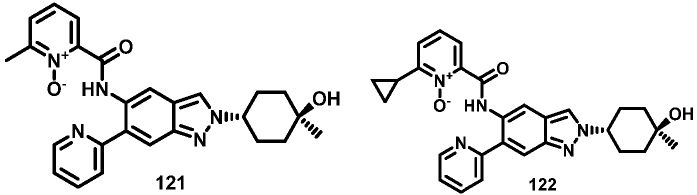

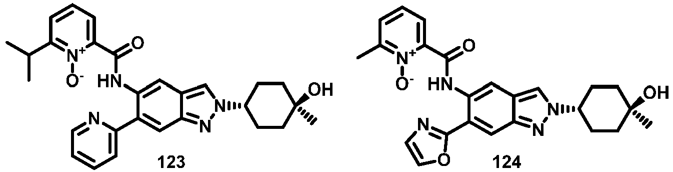

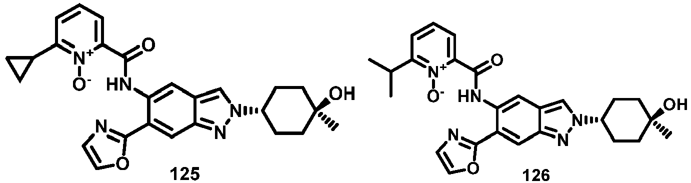

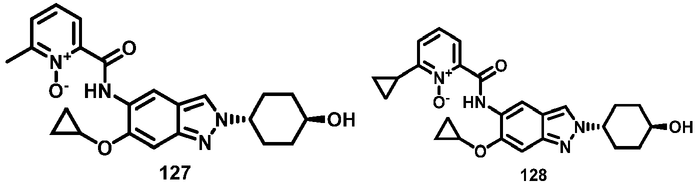

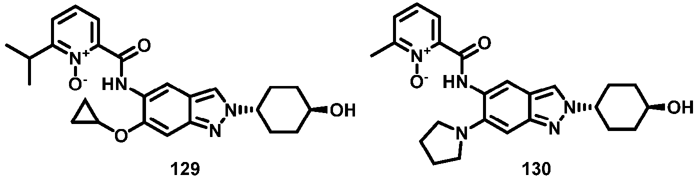

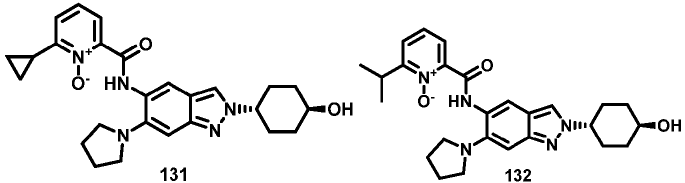

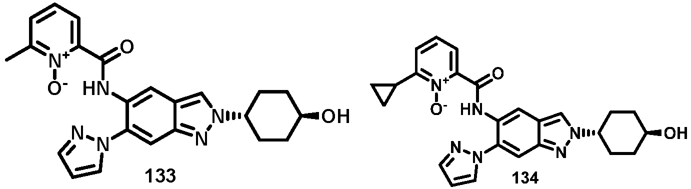

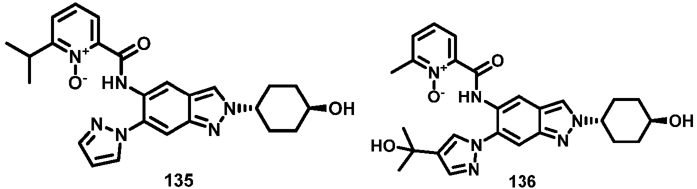

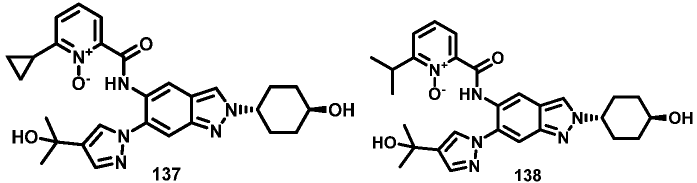

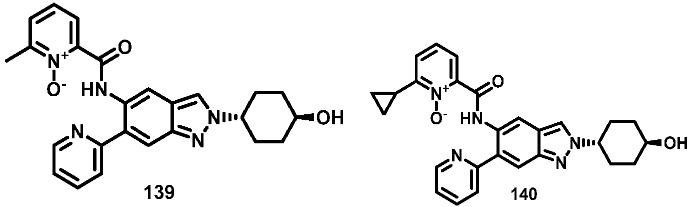

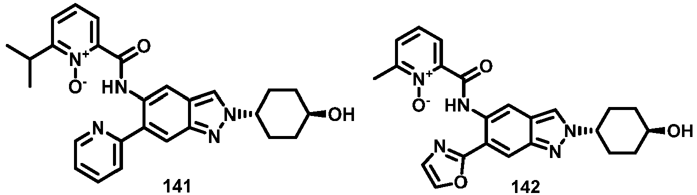

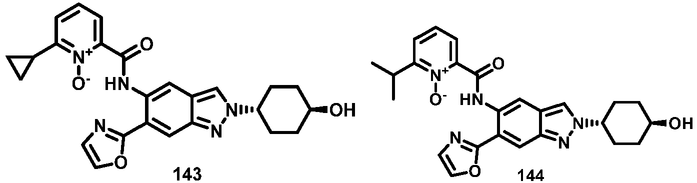

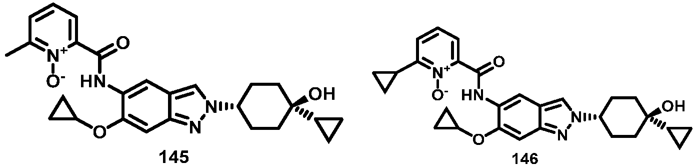

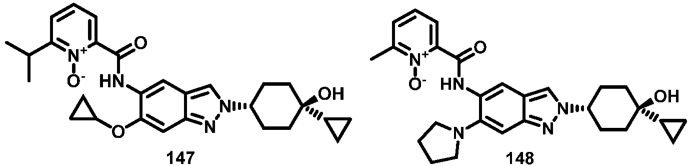

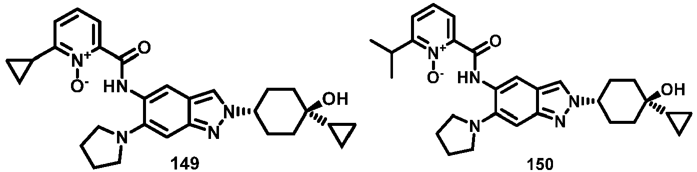

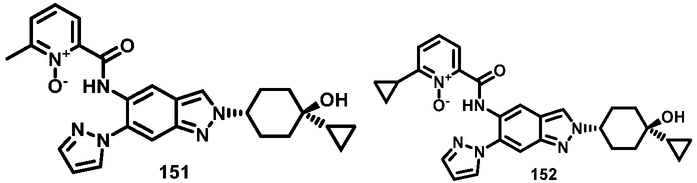

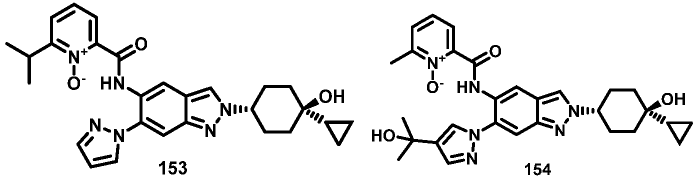

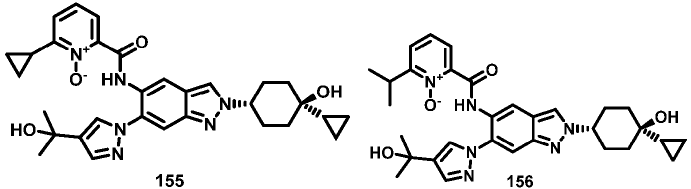

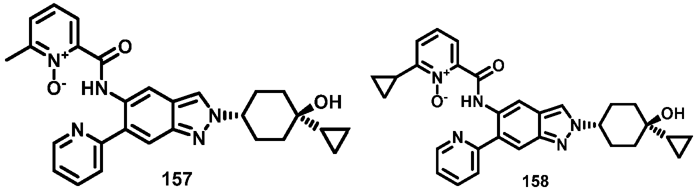

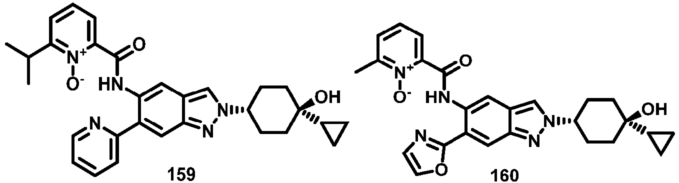

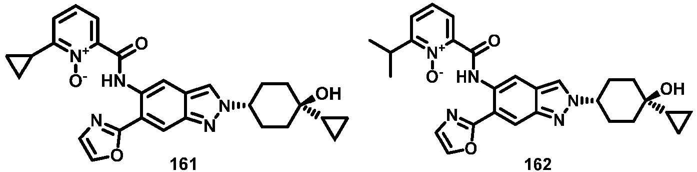

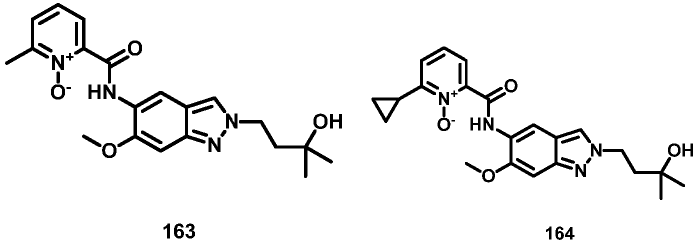

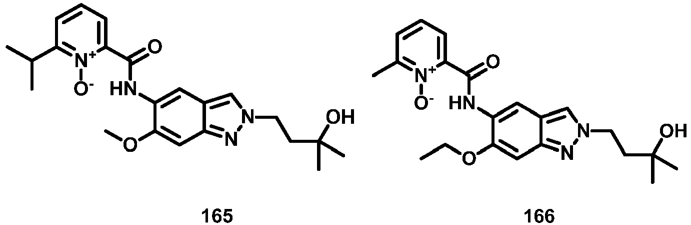

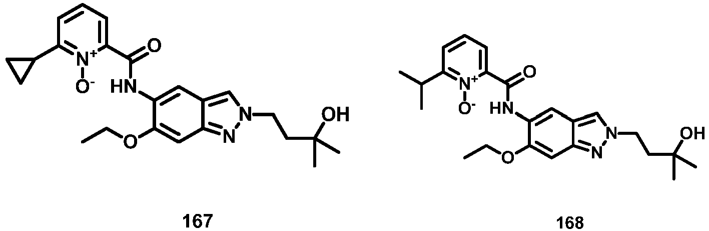

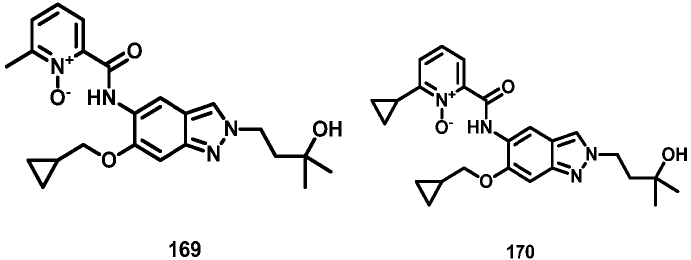

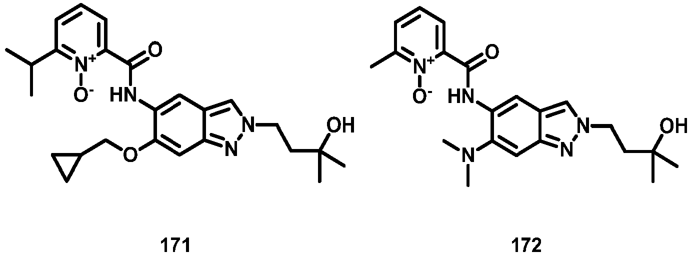

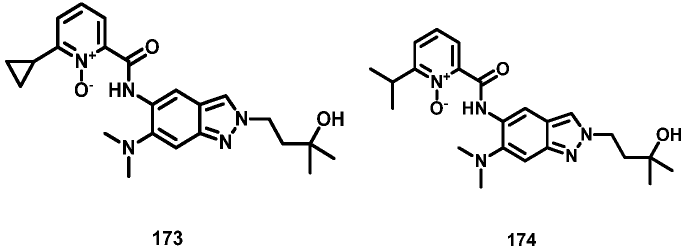

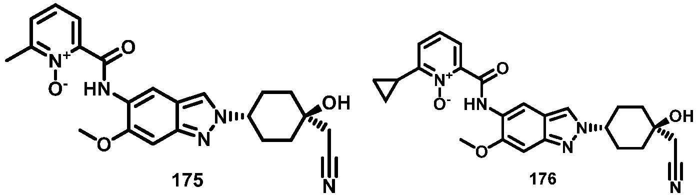

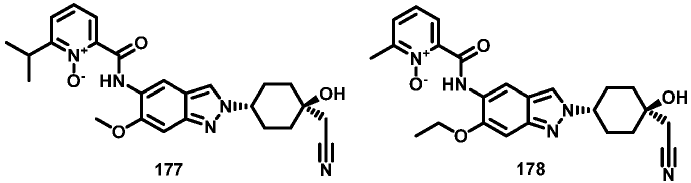

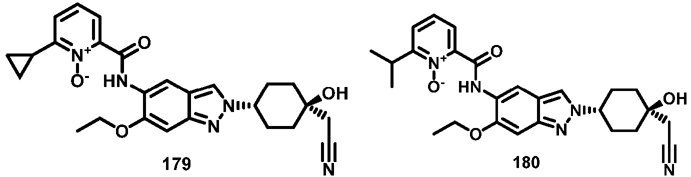

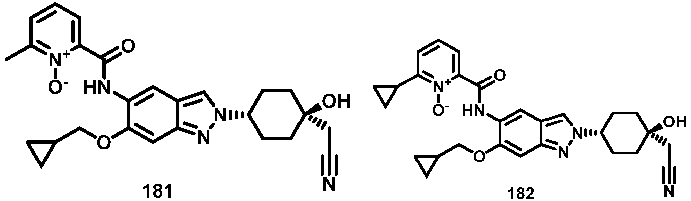

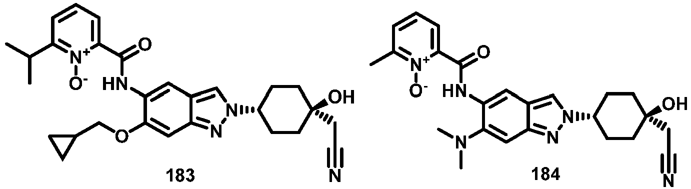

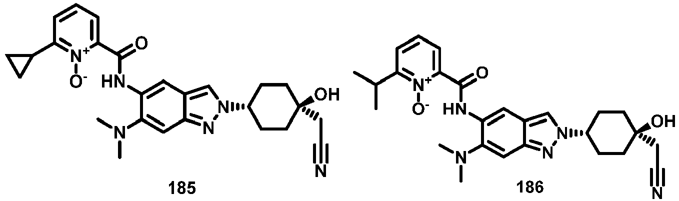

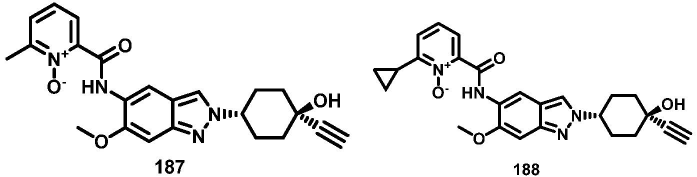

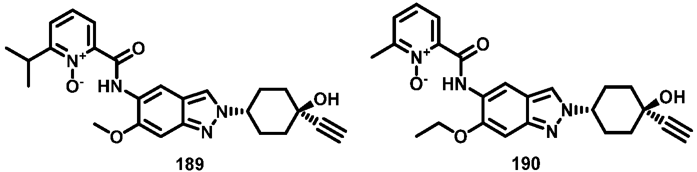

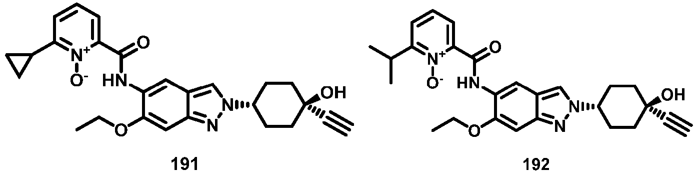

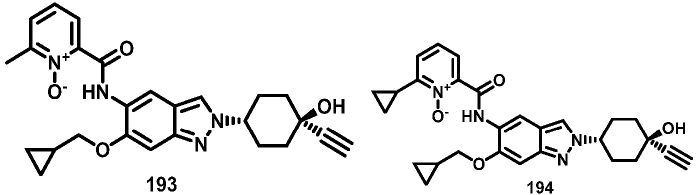

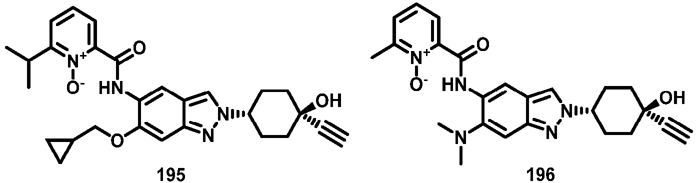

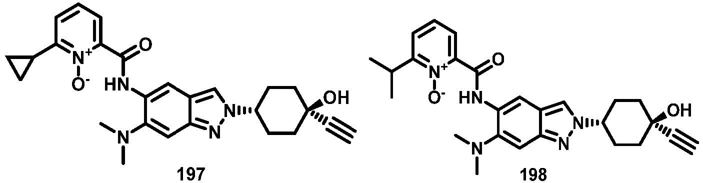

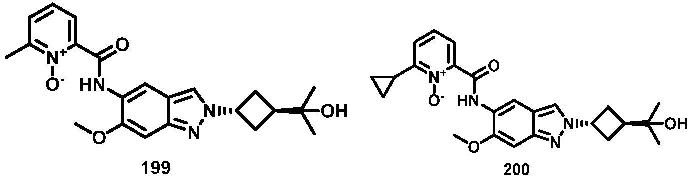

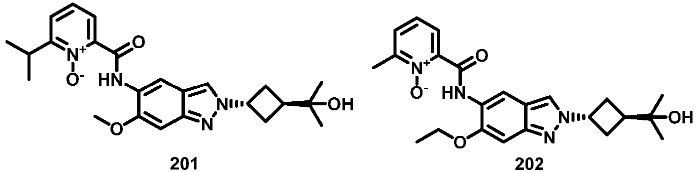

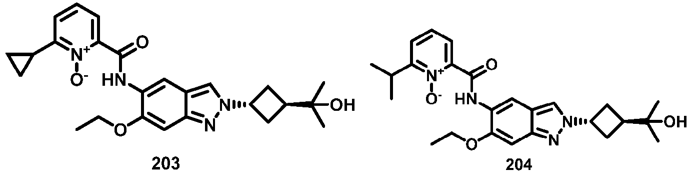

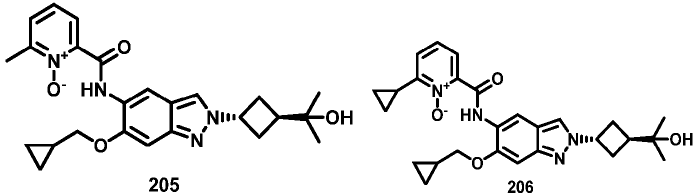

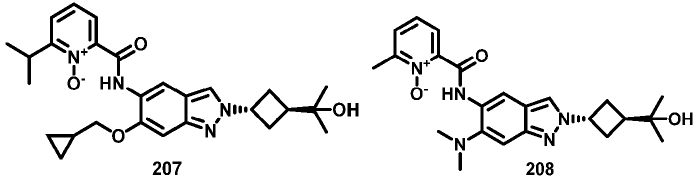

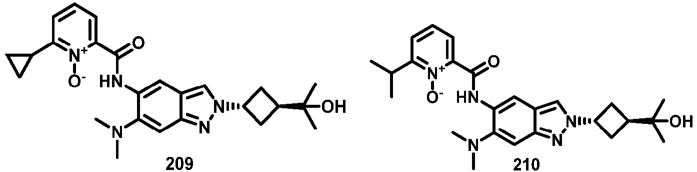

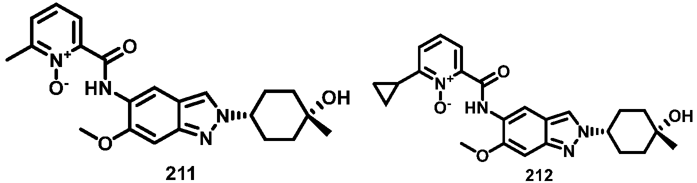

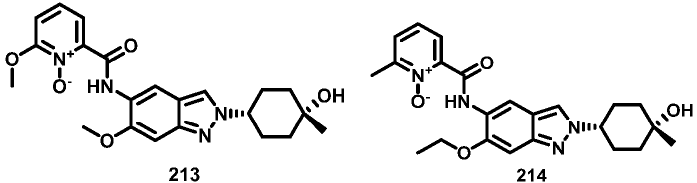

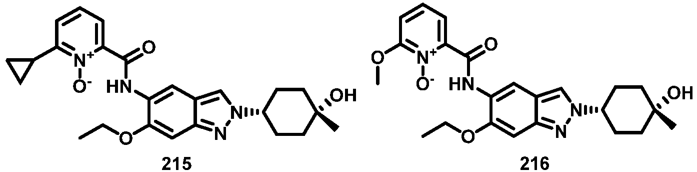

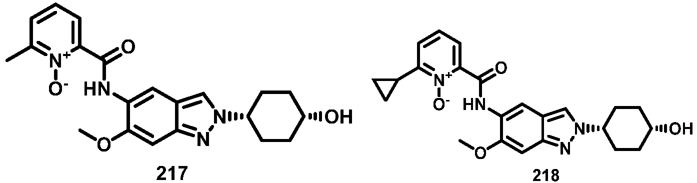

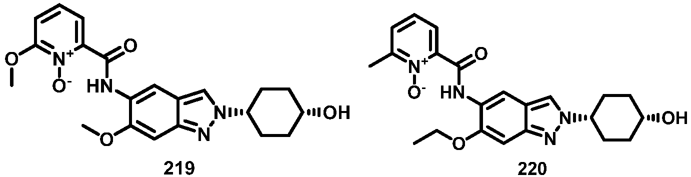

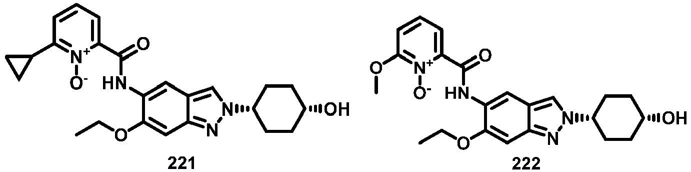

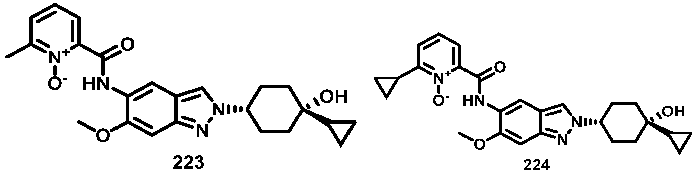

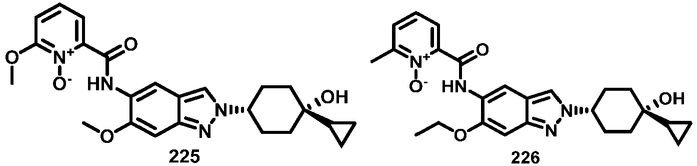

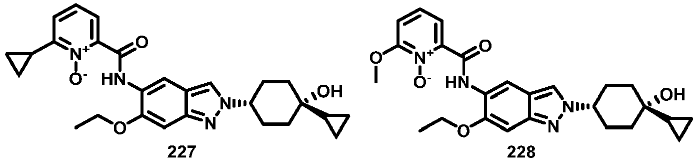

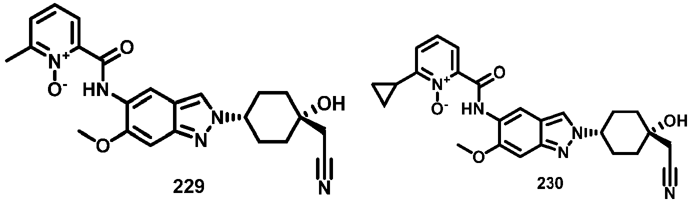

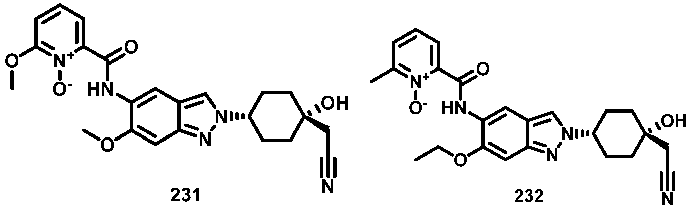

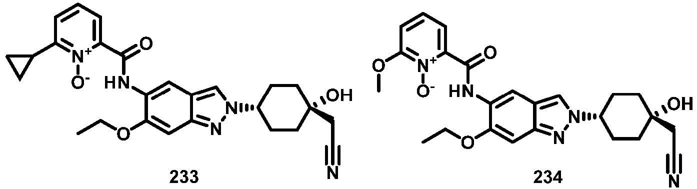

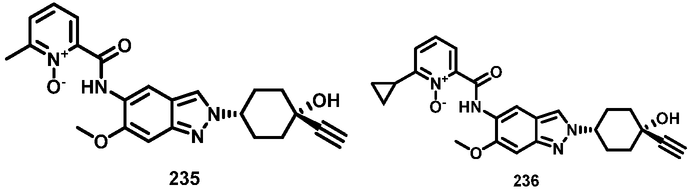

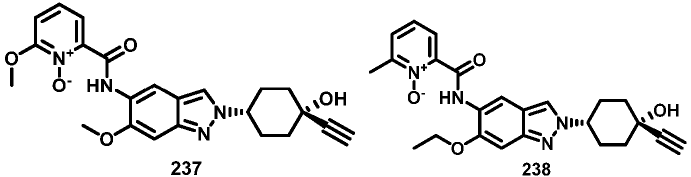

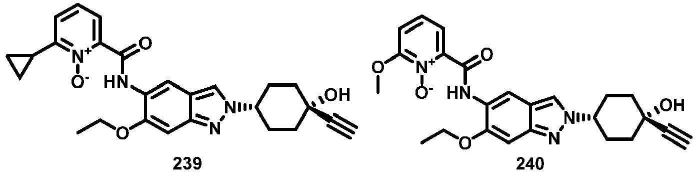

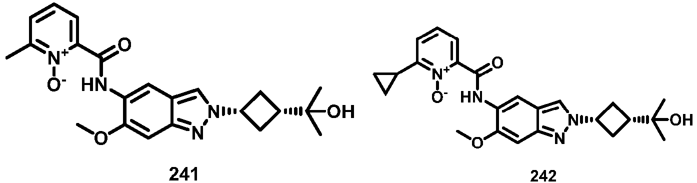

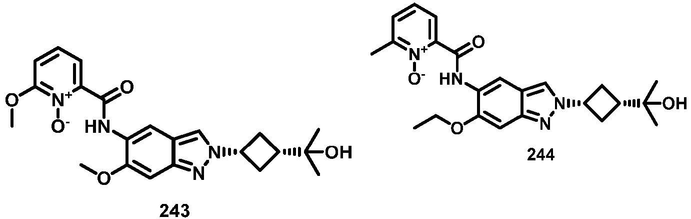

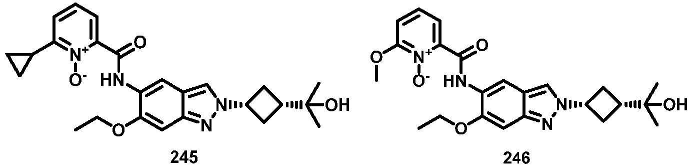

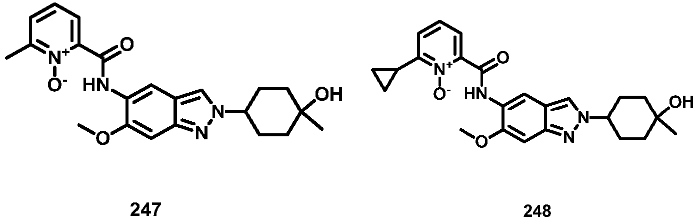

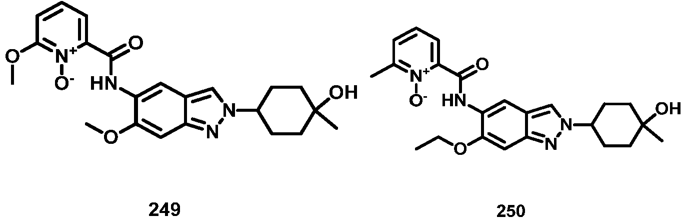

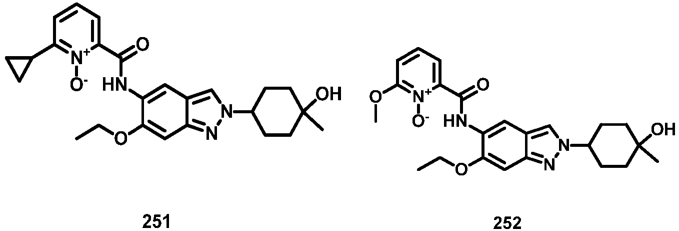

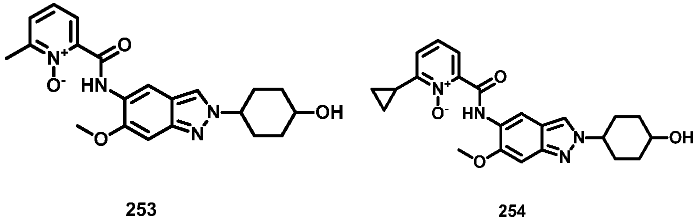

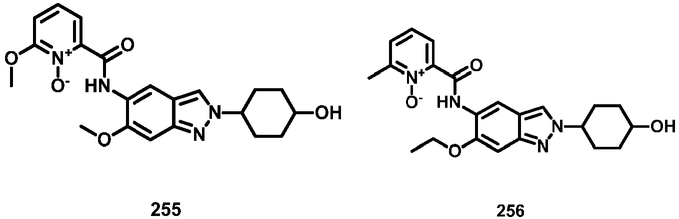

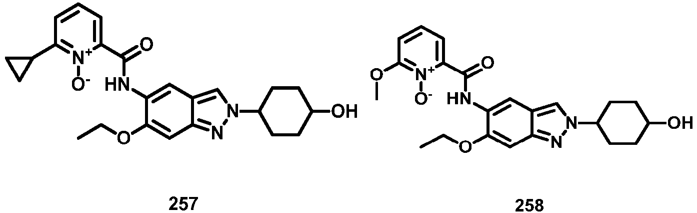

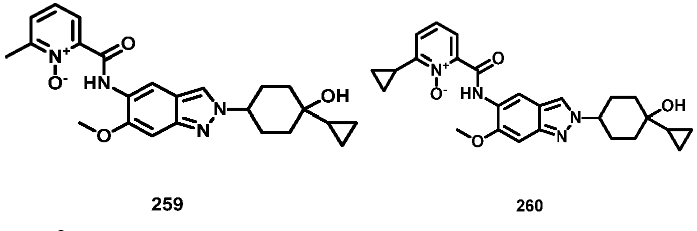

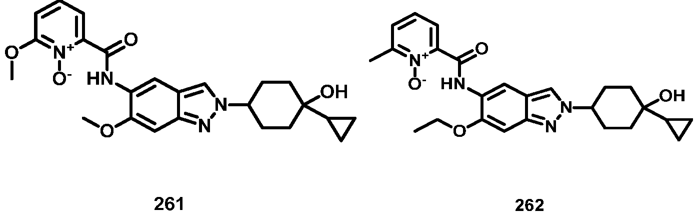

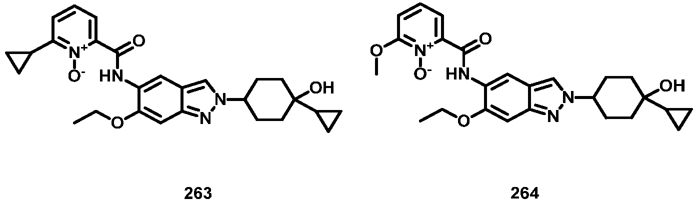

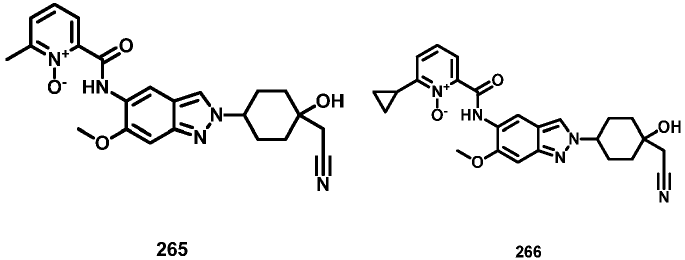

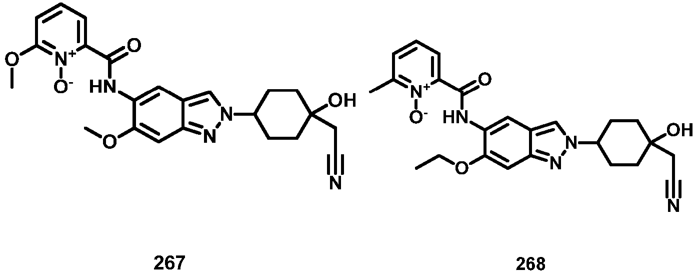

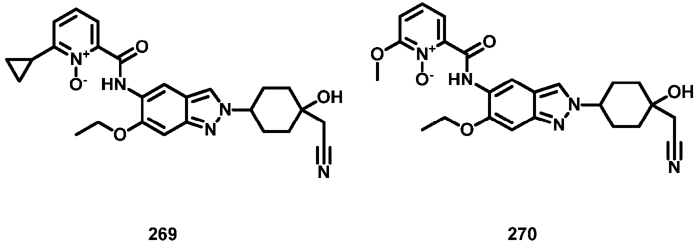

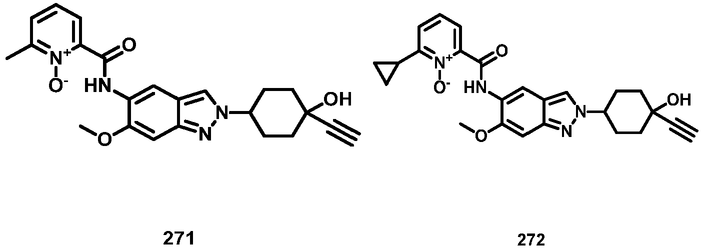

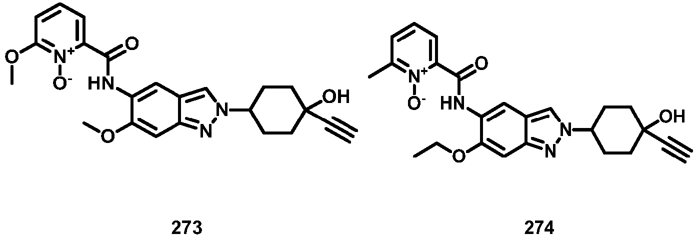

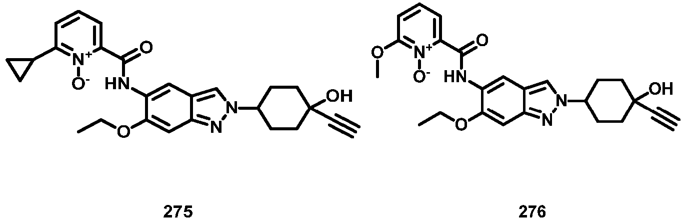

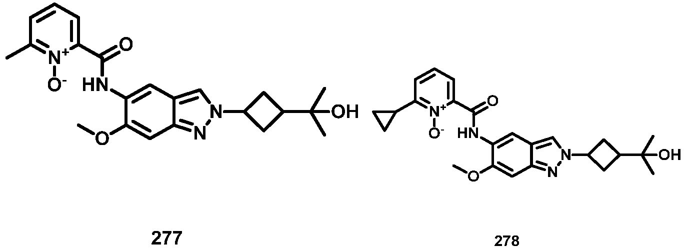

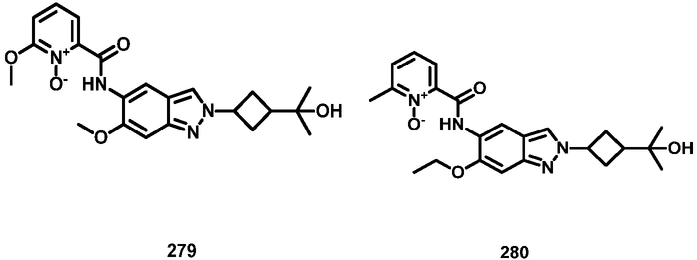

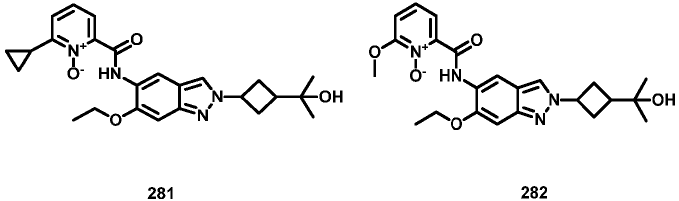

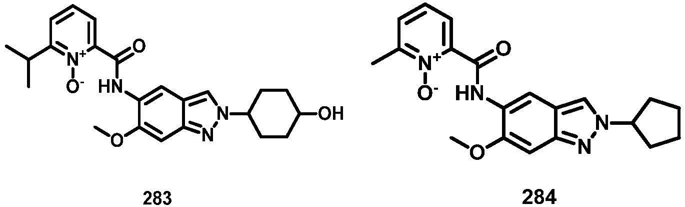

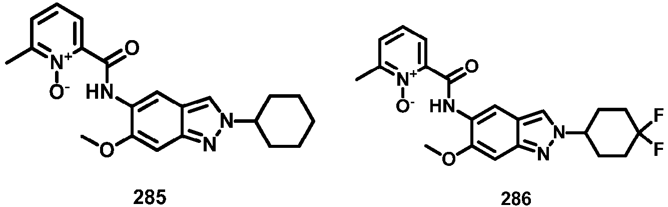

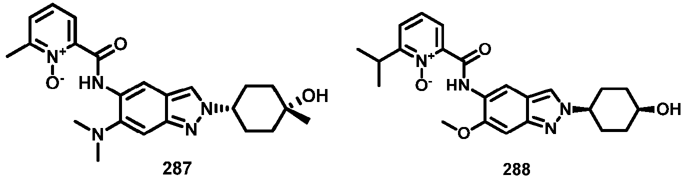

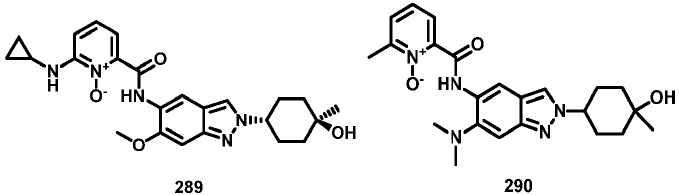

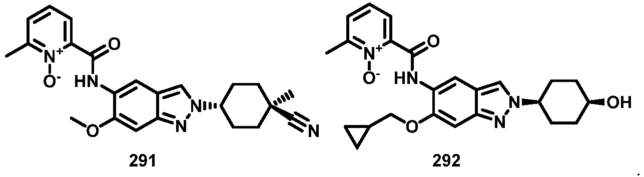

In some embodiments of the present application, the psoriasis is psoriasis vulgaris, psoriatic arthritis, pustular psoriasis, or erythrodermic psoriasis.

In some embodiments, the compound of formula (I), a stereoisomer, a racemate, a tautomer, an isotopically labeled compound, a prodrug, or a pharmaceutically acceptable salt thereof, is administered orally, parenterally, or through other routes of administration. Preferably, the administration is oral or parenteral administration. More preferably, the administration is oral administration.

The parenteral administration may be, for example, topical application to the skin; the other routes of administration include, for example, inhalable pharmaceutical forms, nasal drops, solutions or sprays, tablets, films/oblates or capsules for lingual, sublingual or buccal administration, suppositories, optic or ophthalmic preparations, vaginal capsules, aqueous suspensions, lipophilic suspensions, ointments, creams, transdermal therapeutic systems (e.g., patches), emulsions, pastes, foams, dusting powders, implants, or stents.

The dosage forms for oral administration include tablets, films, capsules, sugar-coated tablets, granules, pills, powders, emulsions, suspensions, or solutions.

In another aspect, the present application provides a method of treating and/or preventing psoriasis, comprising administering a therapeutically effective amount of the compound of formula (I), a stereoisomer, a racemate, a tautomer, an isotopically labeled compound, a prodrug, or a pharmaceutically acceptable salt thereof.

### Beneficial effects

The compounds of the present application have good efficacy in treating and alleviating psoriasis. Specifically, as shown by the experimental studies of the present application, the compounds have shown an improvement in the weight loss of mice, exhibited a significant inhibitory effect on the increase in ear thickness in mice, and exhibited a significant inhibitory effect on spleen enlargement in mice. Furthermore, after treatment with the compounds of the present application, there was a significant reduction in psoriasis symptoms in mice.

### BRIEF DESCRIPTION OF THE DRAWINGS

Fig. 1 shows the changes in the body weight of mice.
Fig. 2 shows the changes in the right ear thickness of mice.
Fig. 3 shows the changes in the dorsal skin scores of mice.
Fig. 4 shows the spleen weight (mg) of mice.
Fig. 5 shows the skin pathology scores of mice.

### DETAILED DESCRIPTION

The technical solutions of the present application are further illustrated in detail in the following examples. It should be understood that the following examples are merely exemplary illustration and explanation of the present application, and should not be construed as limiting the scope of the present application. All techniques implemented based on the contents of the present application are encompassed within the intended scope of the present application.

Unless otherwise specified, the raw materials and reagents used in the following examples are commercially available or can be prepared by known methods.

### Example 1. Preparation of Compounds

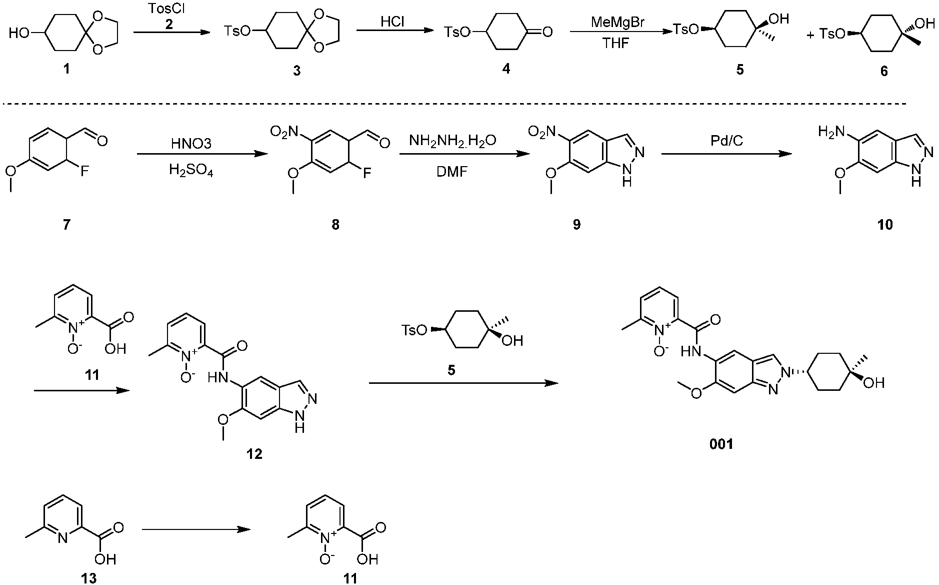

### 1. Synthesis of compound 3

To a solution of compound **1** (50 g) in dichloromethane (500 mL) were sequentially added DMAP (42.5 g), compound **2** (63.4 g), and triethylamine (63.9 g) at 15 °C. The reaction mixture was stirred at 25 °C for 18 hours. The reaction mixture was added with dichloromethane (200 mL) and washed with water (300 mL × 2) and 1 M dilute hydrochloric acid (300 mL × 3). The organic phase was dried over anhydrous sodium sulfate, filtered, and concentrated under reduced pressure to obtain compound 3 (98 g, yield of 99%).

### 2. Synthesis of compound 4

To a solution of compound **3** (50 g) in tetrahydrofuran (300 mL) was added 1 M dilute hydrochloric acid (300 mL) at 15 °C, and the mixture was stirred at 25 °C for 20 hours. The reaction mixture was cooled to 0 °C, added with 1 M sodium hydroxide solution to adjust the pH to 9, and extracted with ethyl acetate (200 mL × 3). The extract was washed with saturated sodium chloride solution (300 mL), dried over anhydrous sodium sulfate, filtered, and concentrated under reduced pressure. The residue was slurried with petroleum ether (150 mL) to obtain compound 4 (39 g, yield of 91%).

### 3. Synthesis of compounds 5 & 6

To a solution of methyl magnesium bromide (85.8 mL) in tetrahydrofuran (500 mL) was added dropwise a solution of compound **4** (34.5 g) in tetrahydrofuran (200 mL) at -40 °C, and the reaction mixture was stirred at -40 °C for 4 hours. The reaction mixture was quenched with saturated ammonium chloride solution (100 mL) and extracted with ethyl acetate (500 mL × 3). The extract was washed with saturated brine (300 mL), dried over anhydrous sodium sulfate, filtered, and concentrated under reduced pressure. The residue was purified by silica gel column chromatography (petroleum ether: ethyl acetate = 5:1) to obtain compound 5 (4.3 g, yield of 10%), compound 6 (7.0 g, yield of 17%), and a mixture (12 g).

### Compound 5

¹H NMR (400 MHz, CDCl₃): δ 7.79 (d, *J =* 8.0 Hz, 2H), 7.32 (d, *J =* 8.4 Hz, 2H), 4.52-4.41 (m, 1H), 2.44 (s, 3H), 1.95-1.80 (m, 2H), 1.77-1.61 (m, 4H), 1.46-1.35 (m, 2H), 1.19 (s, 3H).

### Compound 6

¹H NMR (400 MHz, CDCl₃): δ 7.79 (d, *J =* 8.4 Hz, 2H), 7.33 (d, *J =* 8.0 Hz, 2H), 4.74-4.64 (m, 1H), 2.44 (s, 3H), 1.92-1.79 (m, 2H), 1.77-1.62 (m, 4H), 1.49-1.38 (m, 2H), 1.23 (s, 3H).

### 4. Synthesis of compound 8

To a solution of compound 7 (2.0 g) in concentrated sulfuric acid (12 mL, 98%) was added dropwise a mixed solution of nitric acid (1.6 mL, 70%) in concentrated sulfuric acid (1.6 mL, 98%) at -15 °C. After the addition was completed, the mixed system was stirred at -15 °C for 2 hours. The reaction mixture was then slowly poured into ice water, stirred for 5 minutes, and filtered under reduced pressure. The resulting solid was washed with water, collected, and dried under reduced pressure to obtain compound 8 (2.5 g, yield of 97%).

### 5. Synthesis of compound 9

To a solution of compound **8** (2.0 g) in DMF (20 mL) was added hydrazine hydrate (2.4 mL, 98%) at room temperature. After the addition was completed, the mixed system was heated to 120 °C and stirred for 16 hours. After cooling to room temperature, the mixed system was slowly poured into ice water, stirred, and filtered under reduced pressure. The resulting solid was washed with water, collected, and concentrated under reduced pressure to obtain compound 9 (1.3 g, yield of 67%).

### 6. Synthesis of compound 10

To 400 mL of ethyl acetate were sequentially added compound **9** (12.4 g) and palladium on carbon (7 g, 10%) at 15 °C. After the addition was completed, the mixed system was stirred at 15 °C under a hydrogen atmosphere for 18 hours. After the reaction, the palladium on carbon was filtered off from the reaction mixture, and the filtrate was concentrated to dryness by rotary evaporation to obtain compound 10 (10.4 g, yield of 99%).

### 7. Synthesis of compound 12

To a solution of compound **10** (1.5 g) and compound **11** (1.4 g) in pyridine (15 mL) was added EDCI·HCl (2.6 g) at 25 °C. The reaction mixture was stirred at 25 °C for 16 hours. The reaction mixture was concentrated to dryness by rotary evaporation, and the residue was slurried with a mixture of methanol and water (20 mL/20 mL) to obtain compound 12 (1.3 g, yield of 48%).

### 8. Synthesis of compound 001: 2-((2-(trans-4-hydroxy-cis-4-methylcyclohexyl)-6-methoxy-2H-indazol-5-yl)carbamoyl)-6-methylpyridine 1-oxide

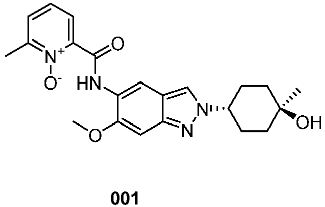

To a solution of compound **12** (300 mg) and compound **5** (344 mg) in DMF (5 mL) was added cesium carbonate (985 mg) at 25 °C. The reaction mixture was stirred at 90 °C for 16 hours. The reaction mixture was added with water (30 mL) and extracted with ethyl acetate (10 mL × 3). The organic phase was concentrated under reduced pressure, and the residue was purified by preparative HPLC (CH₃CN: H₂O (0.1% NH₄HCO₃) = 15 to 45%, UV: 214 nm, flow rate: 15 mL/min) to obtain compound **001** (70 mg, yield of 17%).

¹H NMR (400 MHz, DMSO-*d*₆): δ 14.16 (s, 1H), 8.78 (s, 1H), 8.34 (s, 1H), 8.32-8.30 (m, 1H), 7.77 (d, *J* = 7.6 Hz, 1H), 7.58 (t, *J* = 8.0 Hz, 1H), 7.13 (s, 1H), 4.45 (s, 1H), 4.43-4.40 (m, 1H), 3.95 (s, 3H), 2.53 (s, 3H), 2.09-2.00 (m, 4H), 1.68-1.58 (m, 4H), 1.22 (s, 3H). LCMS: Rt = 3.646 min, [M+H]⁺ = 411.1.

### Biological Example: Psoriasis treatment efficacy of compounds of the present application

### 1.1 Reagents

The compounds of the present application can be prepared with reference to the methods described in Example 1 Preparation of Compounds or described in WO 2021/057785A1.
IMQ (Imiquimod) cream: Aldara
Vaseline: Aladdin
10% neutral formalin fixative: Wuxi Jiangyuan Industrial Technology and Trade Co., Ltd.
Eosin: ZSGB-BIO
Hematoxylin: ZSGB-BIO

### 1.2. Instruments

Micrometer: Mitutoyo, Japan, resolution: 0.001 mm, metric: 0 to 25 mm
LEICA paraffin dehydrator: Leica, model: EG1150C
LEICA paraffin microtome: Leica, model: RM2235
LEICA paraffin embedding station: Leica, model: EG1150H
Upright fluorescence microscope: OLYMPUS, model: DP720

### 1.3 Experimental animals

BALB/c mice, male, 8 weeks old, SPF grade, provided by Shanghai Lingchang Biotechnology Co., Ltd.

Compound A has a structural of

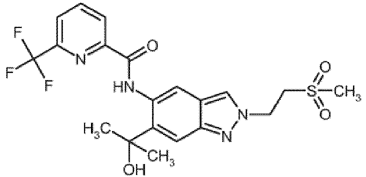

Compound B is compound 001, which has the following structure:

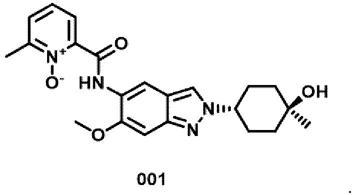

In the drawings:
Group 1 Vaseline-Topical Vehicle P.O indicates a normal control group (vehicle group).
Group 2 IMQ + Vehicle P.O indicates a model control group (vehicle group).
Group 3 IMQ + Dex-Topical indicates a positive control group (dexamethasone).
Group 4 IMQ + A 15 mg/kg P.O indicates a compound group (A, 15 mg/kg, intragastric administration).
Group 5 IMQ + B 15 mg/kg P.O indicates a compound group (B, 15 mg/kg, intragastric administration).

### 1.4 Psoriasis mouse model and administration

First, 50 mice were randomly divided into 5 groups based on body weight: compound group 1 - normal control group (vehicle group); compound group 2 - model control group (vehicle group); compound group 3 - positive control group (dexamethasone); compound group 4 (compound A, 15 mg/kg, intragastrically administered); compound group 5 (compound B, 15 mg/kg, intragastrically administered). On day 0 of the experiment, the dorsal hair of mice in each group was shaved, removing approximately 2 cm * 3 cm area of hair. From day 1 to day 7 of the experiment, except for the normal control group, each mouse received an application of 62.5 mg of 5% IMQ cream on the shaved dorsal area and 20.0 mg of 5% IMQ cream on both sides of the right ear. The normal control group received an application of Vaseline. From day 1 to day 8 of the experiment, two hours before IMQ cream stimulation, the above groups were treated with corresponding drugs. The mice were weighed and shaved prior to administration, which was recorded as day 0. The mice were administered drugs from day 1 to day 8, twice daily.

### 1.5 Test results and evaluation

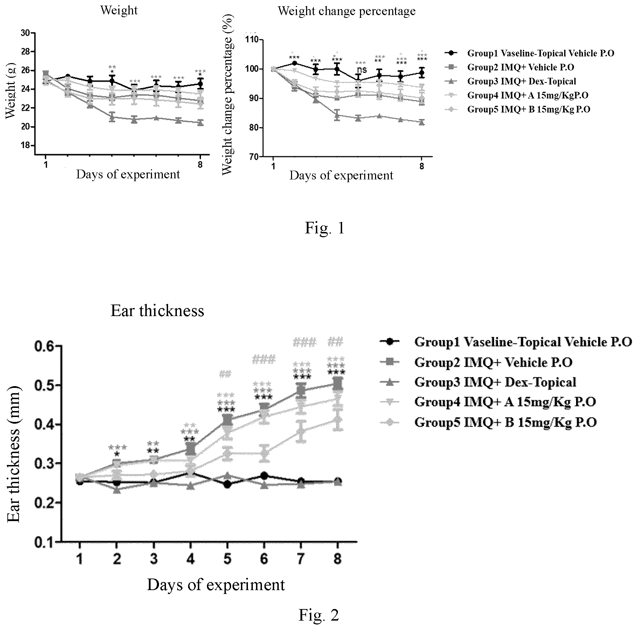

1.5.1. Body weight and rate of change: The body weight was recorded, and the rate of change in body weight was calculated. The results are shown in Fig. 1 (presented as Mean ± SEM, where * P < 0.05, ** P < 0.01, *** P < 0.001, VS. model control group, Two-way ANOVA, Bonferroni posttests, n = 10).

1.5.2. Right ear thickness measurement: The thickness of the right ear of animals in each group was measured and recorded using a micrometer. The results are shown in Fig. 2 (Note: data presented as Mean ± SEM, where * P < 0.05, ** P < 0.01, *** P < 0.001, VS. model control group, Two-way ANOVA, Bonferroni posttests, n = 10; data presented as Mean ± SEM, where # P < 0.5, ## P < 0.01, ### P < 0.001, VS. Group 4, Two-way ANOVA, Bonferroni posttests, n = 10).

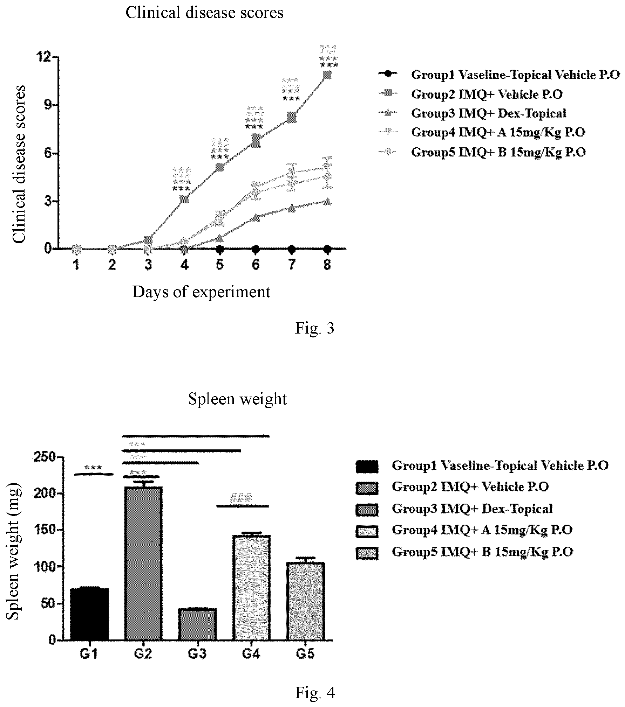

1.5.3. Dorsal skin scoring: On days 1, 3, 5, and 7, a representative photograph was taken for each group, and clinical scoring of the dorsal skin was performed. The clinical scoring criteria are shown in Table 1, and the results are shown in Fig. 3 (data presented as Mean ± SEM, where * P < 0.05, ** P < 0.01, *** P < 0.001, VS. model control group, Two-way ANOVA, Bonferroni posttests, n = 10; data presented as Mean ± SEM, where # P < 0.5, ## P < 0.01, ### P < 0.001, VS. Group 4, Two-way ANOVA, Bonferroni posttests, n = 10).

**Table 1: Clinical scoring system (erythema, crusting, skin thickness)**

| **Score** | **Clinical symptoms** |
|---|---|
| 0 | None |
| 1 | Mild |
| 2 | Moderate |
| 3 | Marked |
| 4 | Extremely marked |

1.5.4. Spleen weight: On day 8, mice in each group were euthanized by CO₂ inhalation, and the spleens were rapidly excised. Right ear and dorsal skin samples were also collected. One-third of the dorsal skin, spleen, and right ear tissues were fixed in 10% neutral formalin fixative for preparation of paraffin sections and HE staining. Another one-third of the dorsal skin was snap-frozen in liquid nitrogen for ELISA analysis. The remaining one-third of the dorsal skin was snap-frozen in liquid nitrogen and stored in a -80 °C freezer for future use. The results are shown in Fig. 4 (Note: data presented as Mean ± SEM, where * P < 0.05, ** P < 0.01, *** P < 0.001, V.S. model control group, One-way ANOVA, Bonferroni posttests, n = 10; data presented as Mean ± SEM, where # P < 0.5, ## P < 0.01, ### P < 0.001, V.S. Group 4, One-way ANOVA, Bonferroni posttests, n = 10).

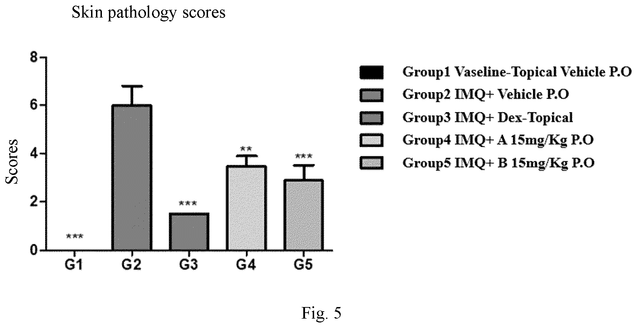

1.5.5. Dorsal skin pathology scoring: Dorsal skin tissue samples were collected from mice and fixed in 10% neutral formalin fixative for 48 hours. The tissues were then degreased in 75% ethanol for 72 hours, with daily medium changes. Dehydration was performed using a graded ethanol series (see Table 2 for details), followed by preparation into paraffin samples. Sections of 4 µm thickness were cut using a rotary microtome and dried in an oven at 60 °C for 2 hours. HE staining and scoring were conducted according to Tables 2 and 3. The results are shown in Fig. 5 (Note: data presented as Mean ± SEM, where * P < 0.05, ** P < 0.01, *** P < 0.001, V.S. model control group, One-way ANOVA, Bonferroni posttests, n = 10).

**Table 2: Skin tissue dehydration steps**

| **Step** | **Reagent** | **Time (hour:minute)** | **Temperature** |
|---|---|---|---|
| **1** | 80% Ethanol | 00:45 | Room temperature |
| **2** | 95% Ethanol | 00:45 | Room temperature |
| **3** | 95% Ethanol | 00:45 | Room temperature |
| **4** | 100% Ethanol | 00:45 | Room temperature |
| **5** | 100% Ethanol | 00:45 | Room temperature |
| **6** | 100% Ethanol | 00:45 | Room temperature |
| **7** | 100% Ethanol | 00:45 | Room temperature |
| **8** | 100% Ethanol | 00:45 | Room temperature |
| **9** | Xylene | 01:00 | Room temperature |
| **10** | Xylene | 01:00 | Room temperature |
| **11** | Paraffin | 01:00 | 60 °C |
| **12** | Paraffin | 01:00 | 60 °C |
| **13** | Paraffin | 01:00 | 60 °C |

**Table 3: H.E. staining steps**

| **Step** | **Reagent** | **Time (minute:second)** | **Step** | **Reagent** | **Time (minute:second)** |
|---|---|---|---|---|---|
| **1** | Xylene | 5:00 | **12** | Water wash 3 | 2:00 |
| **2** | Xylene | 5:00 | **13** | Water wash 4 | 15:00 |
| **3** | Xylene | 5:00 | **14** | 80% Ethanol | 2:00 |
| **4** | 100% Ethanol | 5:00 | **15** | Eosin staining solution | 0:10 |
| **5** | 100% Ethanol | 5:00 | **16** | 95% Ethanol | 0:15 |
| **6** | 95% Ethanol | 5:00 | **17** | 95% Ethanol | 0:15 |
| **7** | 80% Ethanol | 5:00 | **18** | 100% Ethanol | 1:00 |
| **8** | Water wash 1 | 2:00 | **19** | 100% Ethanol | 1:00 |
| **9** | Hematoxylin staining solution | 3:00 | **20** | 100% Ethanol | 1:00 |
| **10** | Water wash 2 | 2:00 | **21** | Xylene | 3:00 |
| **11** | 1% hydrochloric acid in ethanol | 0:05 | **22** | Xylene | 3:00 |
| | | | **EXIT** | Xylene | 3:00 |

In summary, according to Figs. 1 to 5, compared to the model control group, the use of the compounds of the present application (see compound group 5) significantly inhibited the weight loss, the increase in right ear thickness, and the increase in spleen weight in mice, resulting in a significant reduction in psoriasis symptoms, a significant reduction in PASI clinical scores, as well as alleviation of disease symptoms in the keratin, epidermis, and dermis of the dorsal skin of mice, with a significant reduction in pathological scores.

The above descriptions pertain to the specific examples of the present application. It should be understood that the present application is not limited to the aforementioned examples. The examples and descriptions provided are solely to illustrate the principles of the present application. Various non-substantial changes and improvements may be made by those skilled in the art without departing from the concept of the present application, all of which fall within the scope of protection sought by the present application.

## Claims

1. Use of a compound in the manufacture of a medicament for treating and/or preventing psoriasis, wherein the compound is a compound of formula (I): 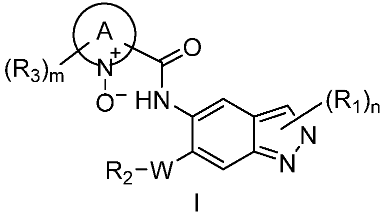 or a stereoisomer, a racemate, a tautomer, an isotopically labeled compound, a prodrug, or a pharmaceutically acceptable salt thereof, wherein:
ring Ais 5- to 14-membered heteroaryl or 5- to 12-membered heterocyclyl comprising at least one N;
R₁, R₂, and R₃ are each independently selected from hydrogen, halogen, CN, OH, or the following groups: (C₁-C₁₂) aliphatic hydrocarbyl, (C₁-C₁₂) aliphatic hydrocarbyl optionally containing one, two, or more heteroatoms, C₃-₁₂ cycloalkyl, 3- to 12-membered heterocyclyl, C₆₋₂₀ aryl, 5- to 14-membered heteroaryl, or -NRₐR_{b}; each of which is optionally substituted by one, two, or more R;
W is selected from O, S, NH, or a single bond;
Rₐ and R_{b} are each independently selected from H or (C₁-C₁₂) aliphatic hydrocarbyl;
each R is independently selected from halogen, CN, OH, SH, NRₐR_{b}, or the following groups: (C₁-C₁₂) aliphatic hydrocarbyl, (C₁-C₁₂) aliphatic hydrocarbyl optionally containing one, two, or more heteroatoms, C₃-₁₂ cycloalkyl, 3- to 12-membered heterocyclyl, C₆₋₂₀ aryl, or 5- to 14-membered heteroaryl; each of which is optionally substituted with one, two, or more R';
each R' is independently selected from halogen, CN, OH, SH, or NRₐR_{b};
n is selected from 1, 2, or 3;
m is selected from 1, 2, 3, 4, 5, or 6;
the "(C₁-C₁₂) aliphatic hydrocarbyl optionally containing one, two, or more heteroatoms" is optionally selected from (C₁-C₁₂) aliphatic hydrocarbyloxy, (C₁-C₁₂) aliphatic hydrocarbylthio, (C₁-C₆) aliphatic hydrocarbyloxy-(C₁-C₆) aliphatic hydrocarbyl, (C₁-C₆) aliphatic hydrocarbylthio-(C₁-C₆) aliphatic hydrocarbyl, N-(C₁-C₃) aliphatic hydrocarbylamino-(C₁-C₆) aliphatic hydrocarbyl, or N, N-di-(C₁-C₃) aliphatic hydrocarbylamino-(C₁-C₆) aliphatic hydrocarbyl;
the "5- to 14-membered heteroaryl or 5- to 12-membered heterocyclyl comprising at least one N" is optionally a heteroaryl or heterocyclyl comprising at least one nitrogen atom on the ring, and optionally further comprising additional heteroatoms selected from N, O, or S, such as selected from pyridine, pyrrole, piperidine, or tetrahydropyrrole;
the (C₁-C₁₂) aliphatic hydrocarbyl is optionally selected from (C₁-C₁₂) alkyl, (C₂-C₁₂) alkenyl, or (C₂-C₁₂) alkynyl; preferably, each (C₁-C₁₂) aliphatic hydrocarbyl is selected from (C₁-C₆) alkyl, (C₂-C₆) alkenyl, or (C₂-C₆) alkynyl;
the "halogen" is optionally selected from F, Cl, Br, or I;
the "C₃₋₁₂ cycloalkyl" is optionally selected from cyclopropyl, cyclobutyl, cyclopentyl, or cyclohexyl.

2. The use according to claim 1, wherein the compound of formula I is a compound of formula Ia, formula Ib, formula Ic, formula Id, or formula Ie: 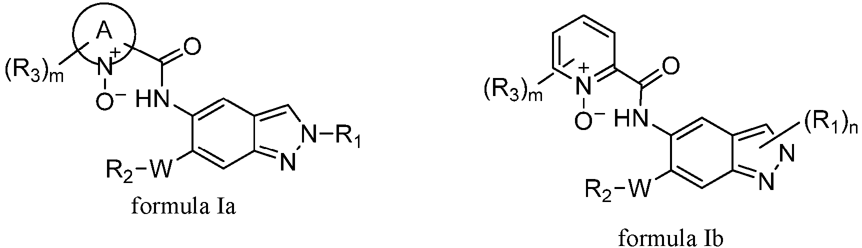 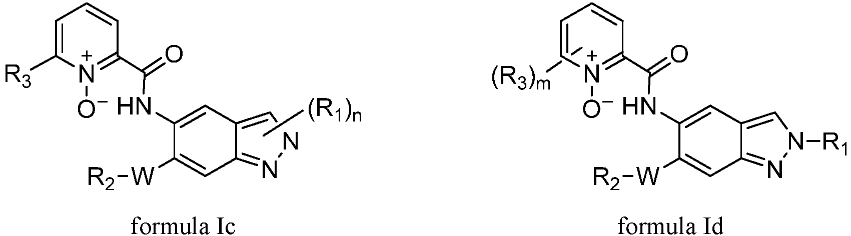 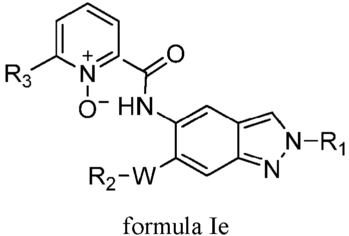 wherein in formula Ia, formula Ib, formula Ic, formula Id, and formula Ie, R₁, R₂, R₃, m, n, and W are as defined in formula I.

3. The use according to claim 1, wherein the compound of formula I is selected from the following structures:

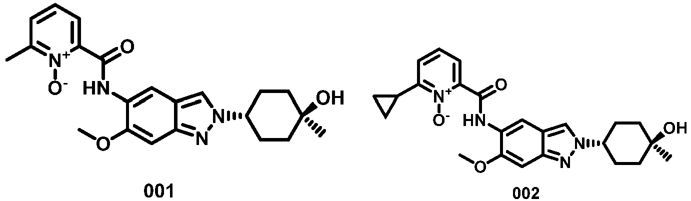

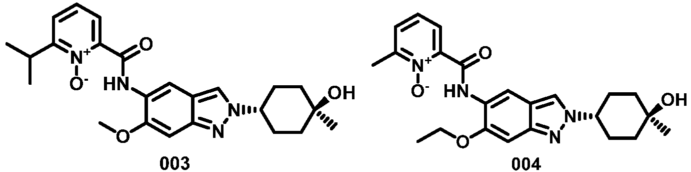

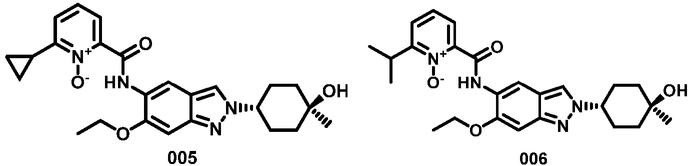

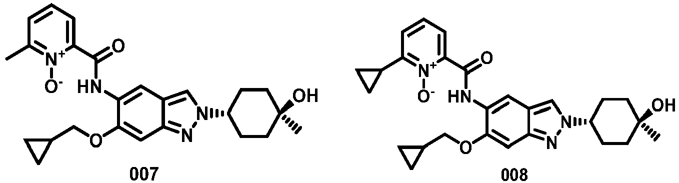

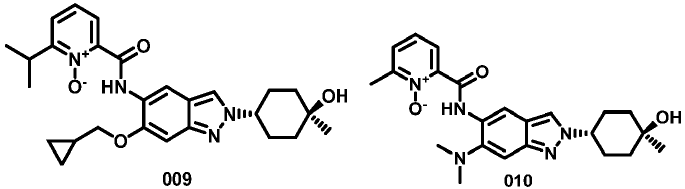

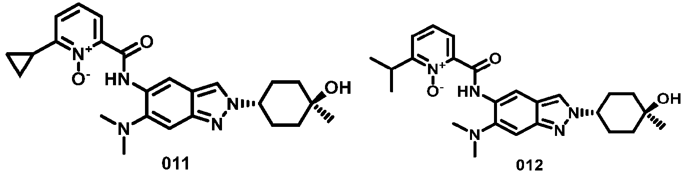

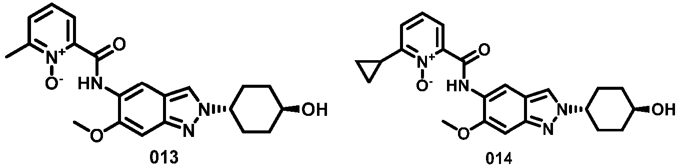

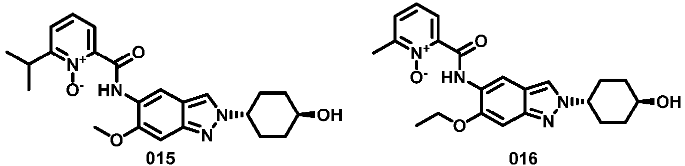

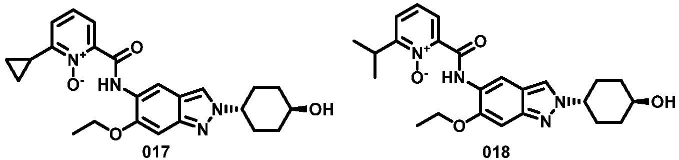

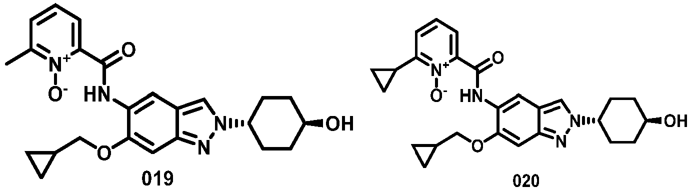

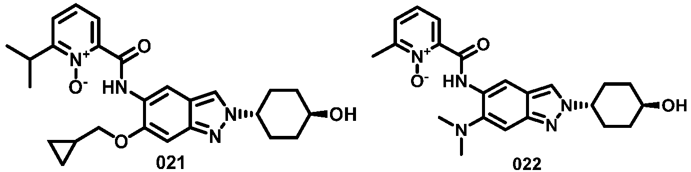

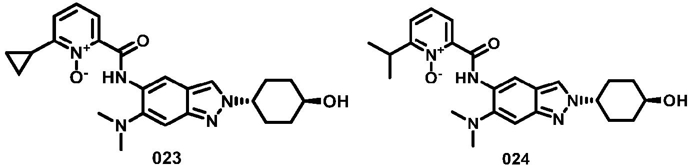

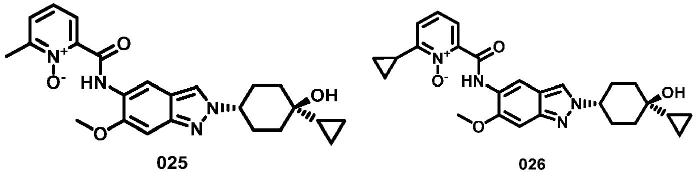

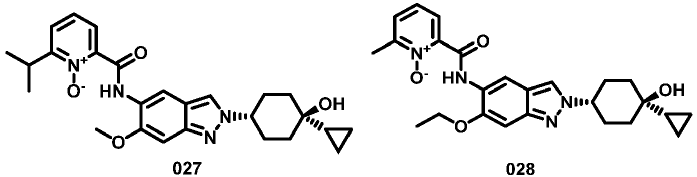

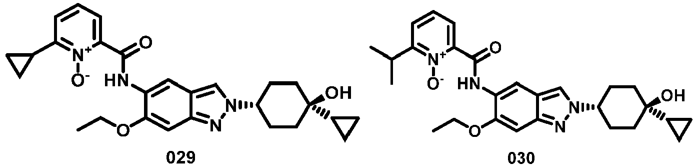

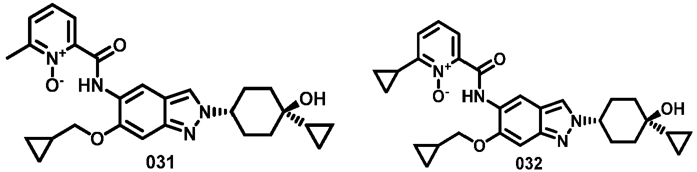

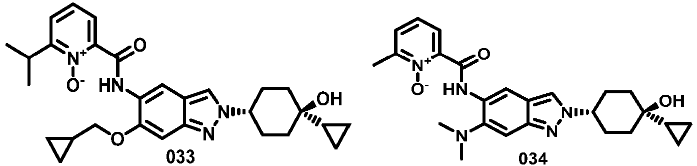

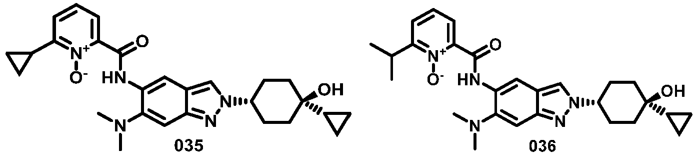

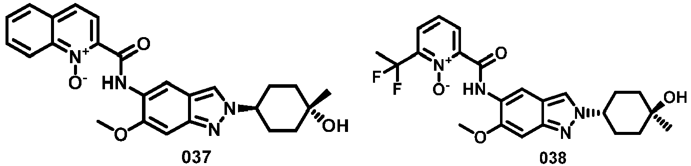

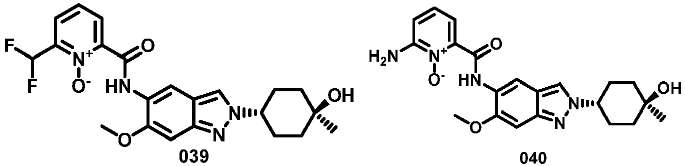

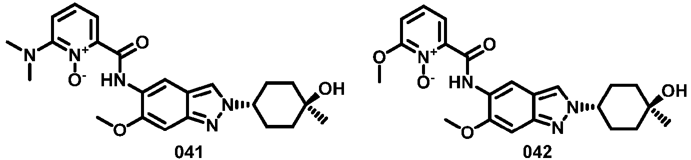

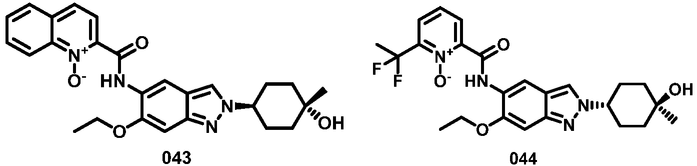

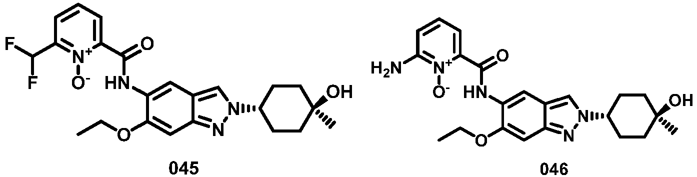

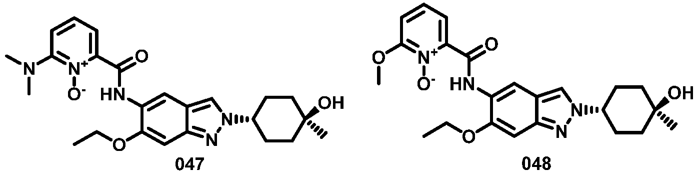

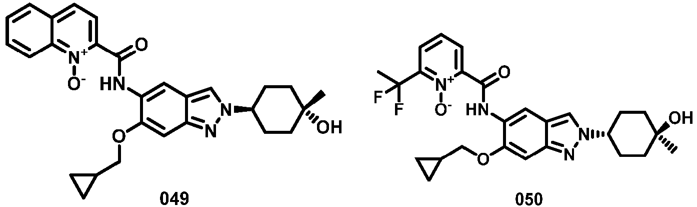

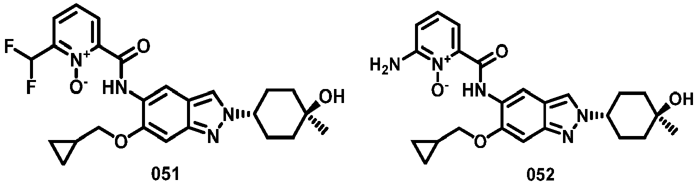

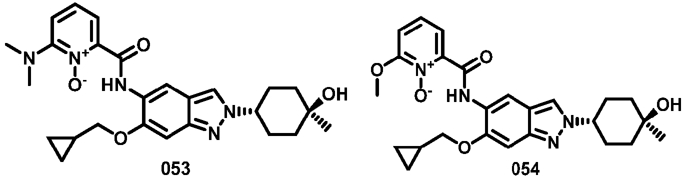

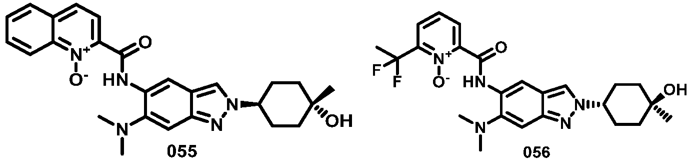

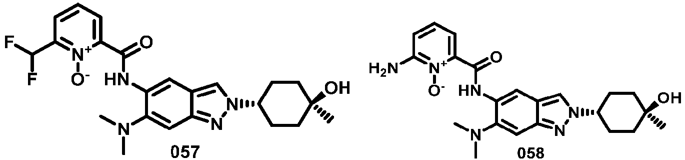

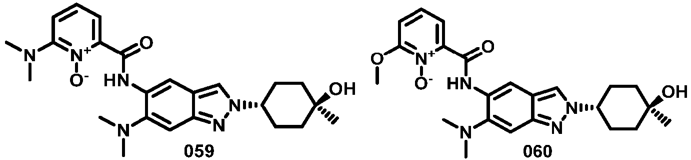

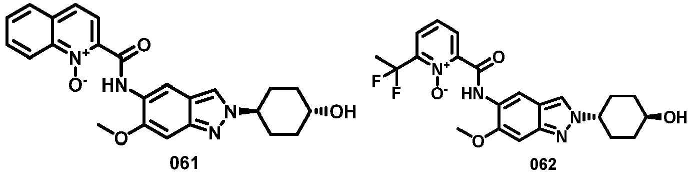

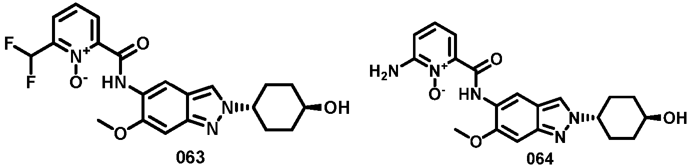

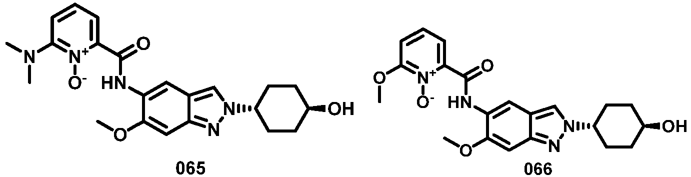

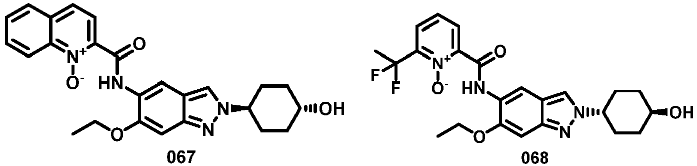

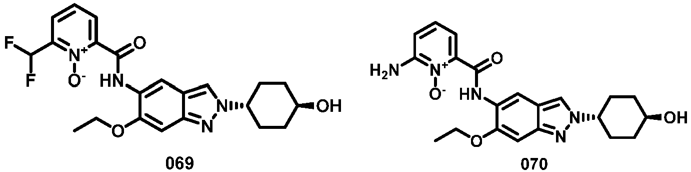

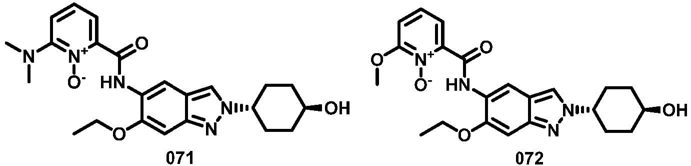

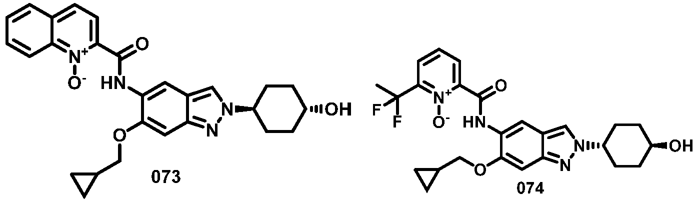

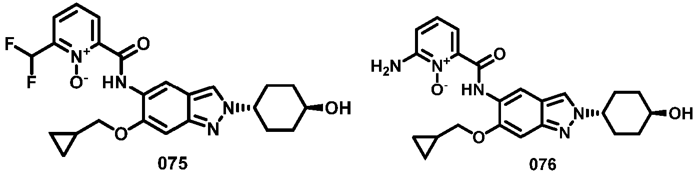

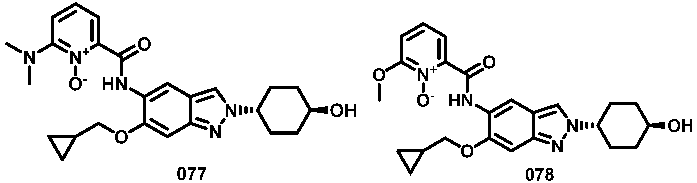

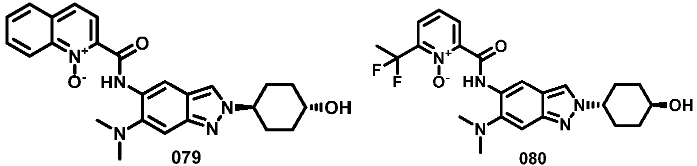

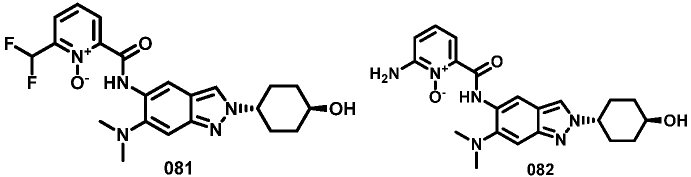

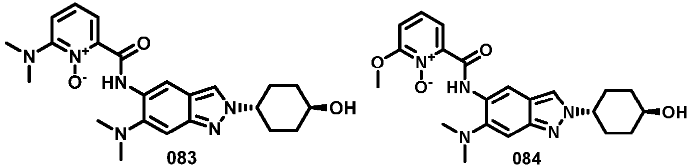

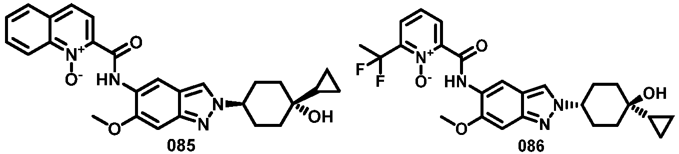

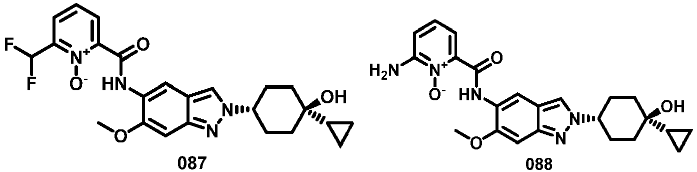

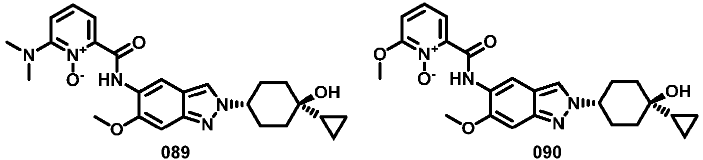

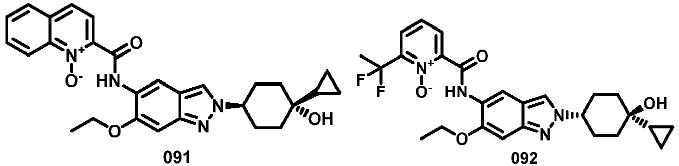

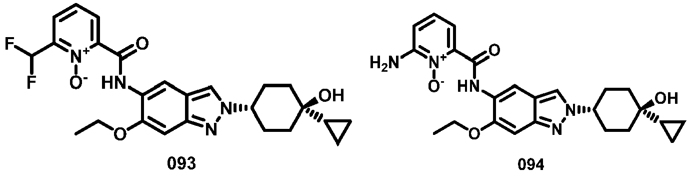

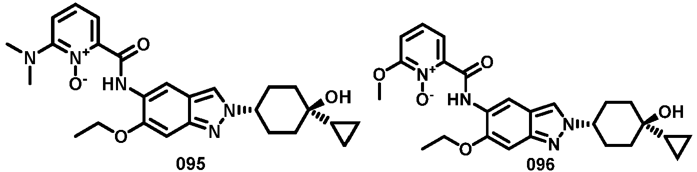

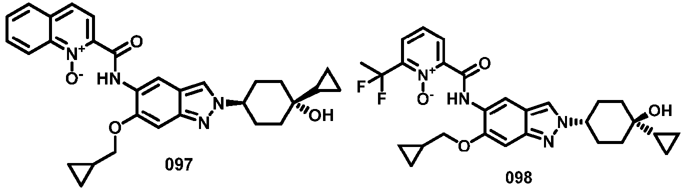

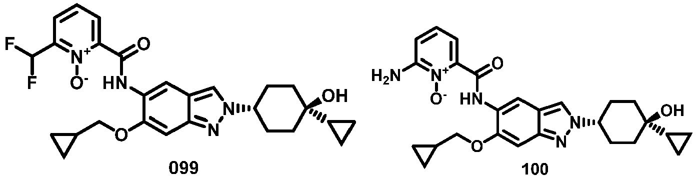

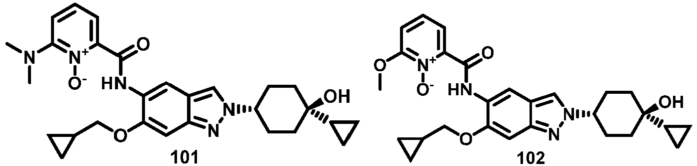

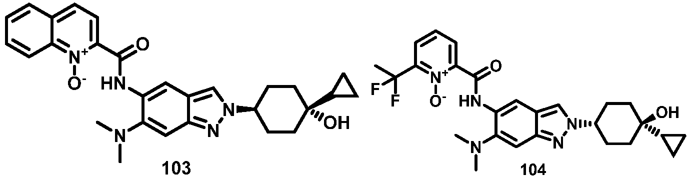

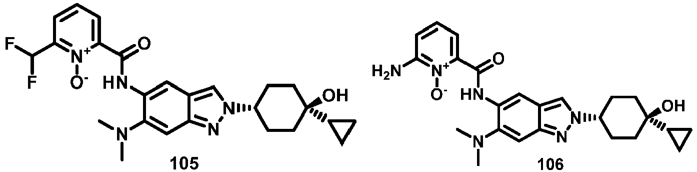

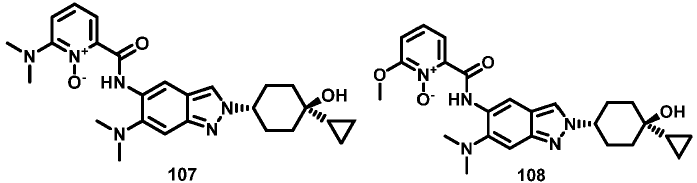

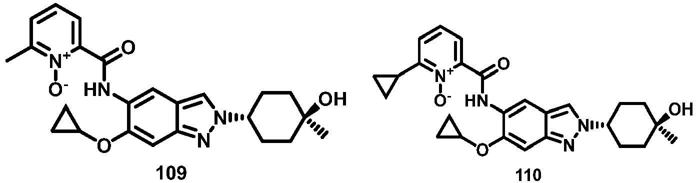

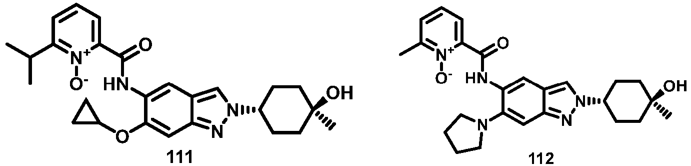

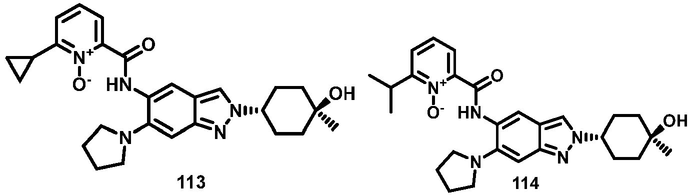

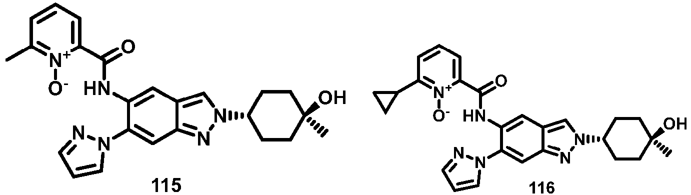

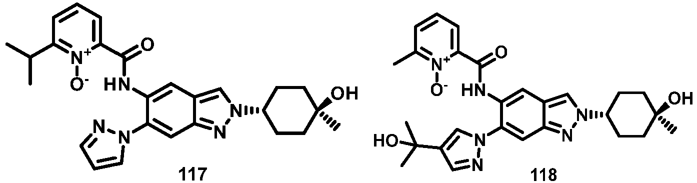

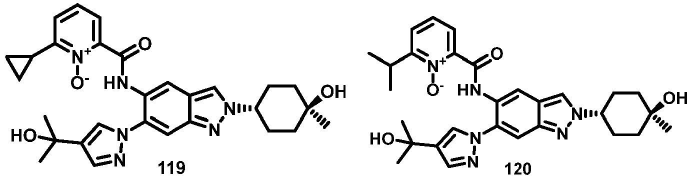

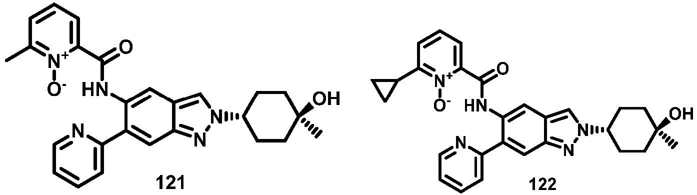

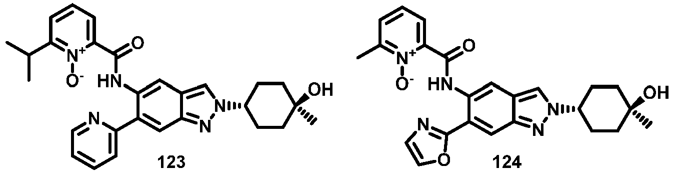

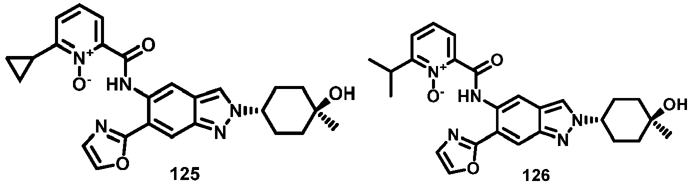

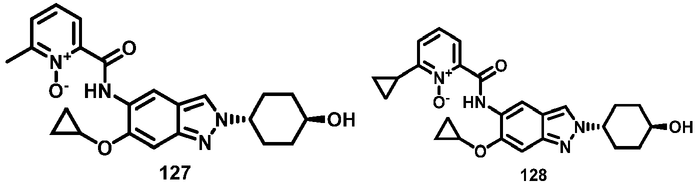

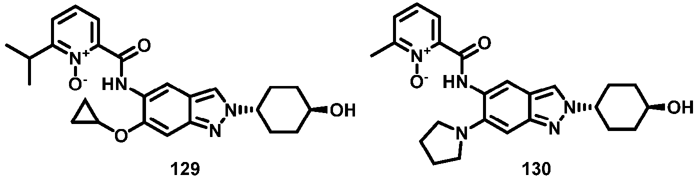

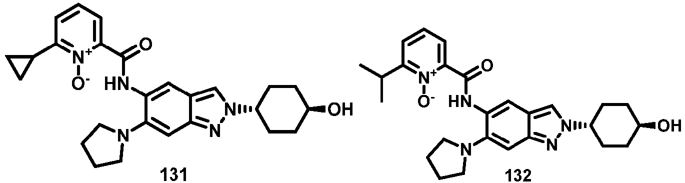

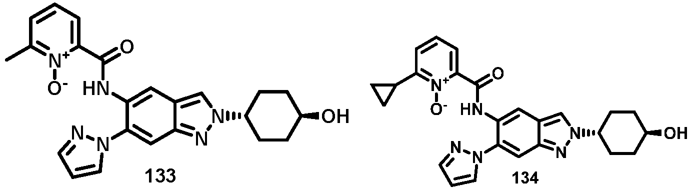

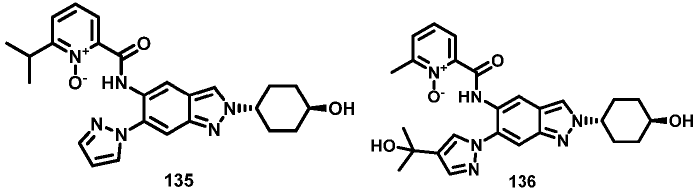

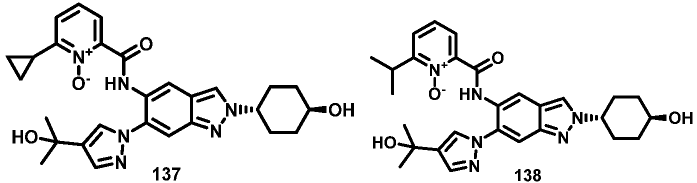

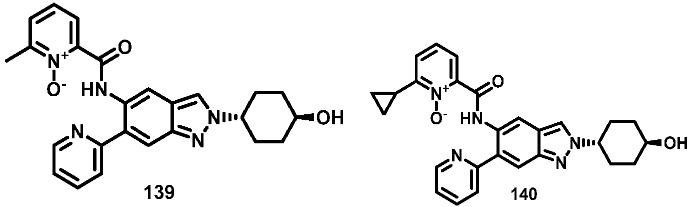

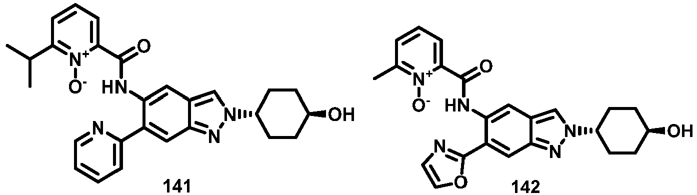

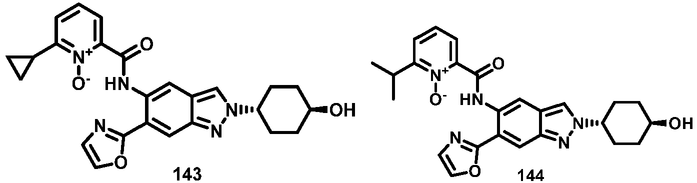

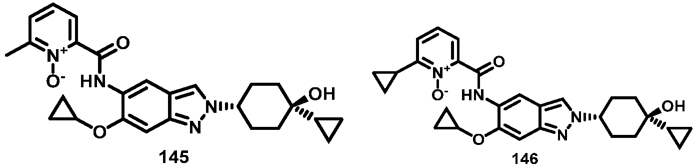

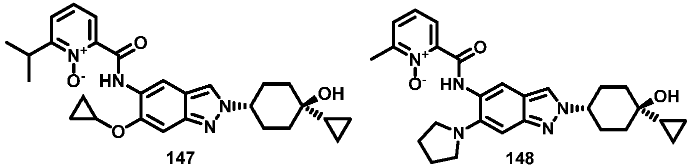

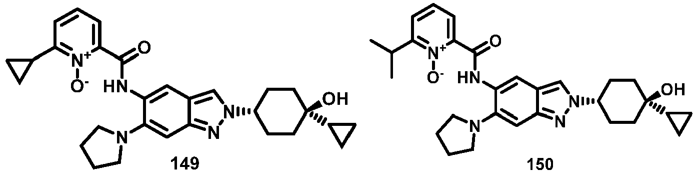

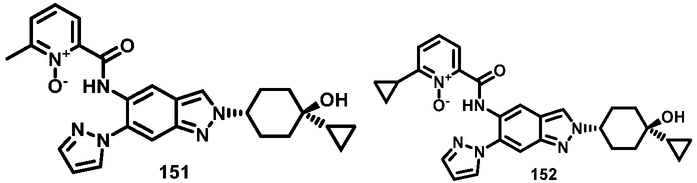

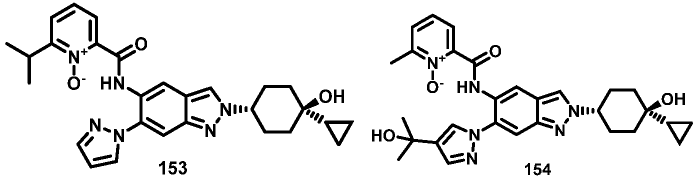

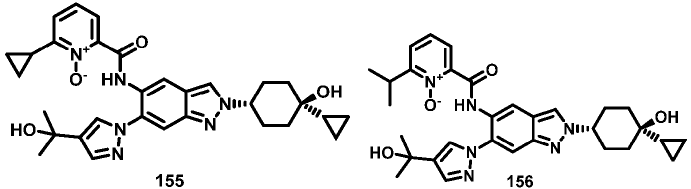

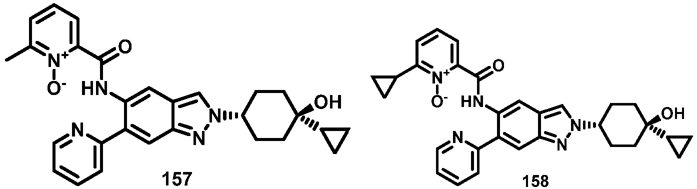

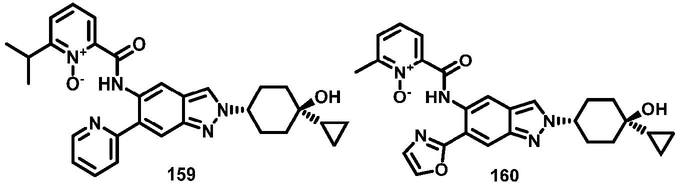

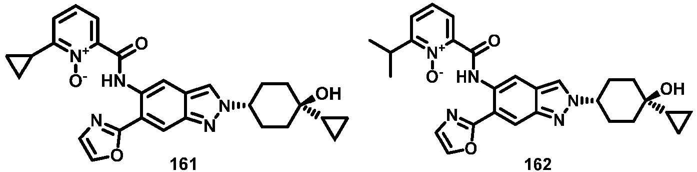

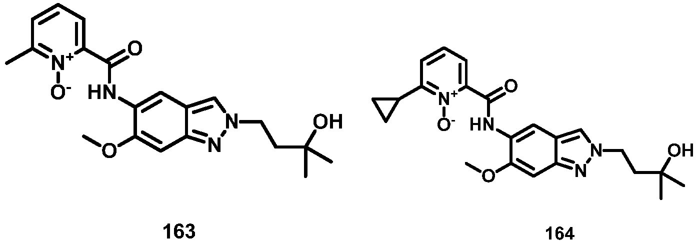

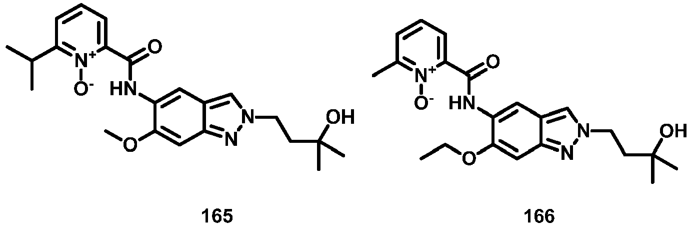

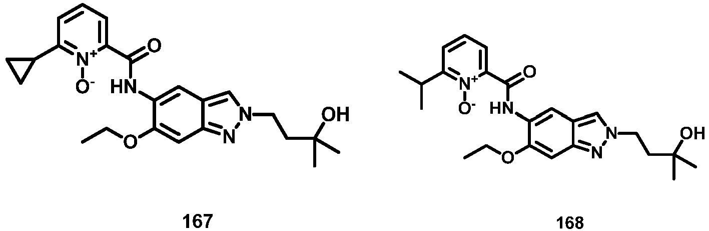

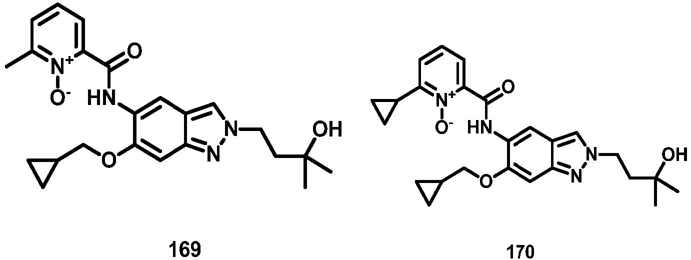

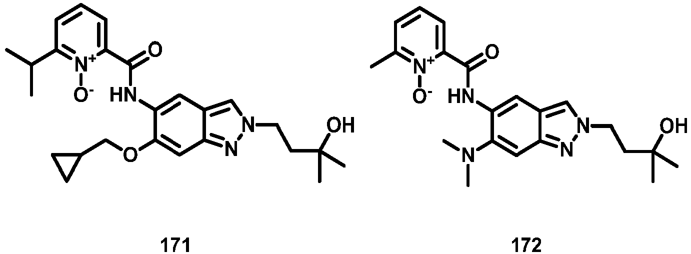

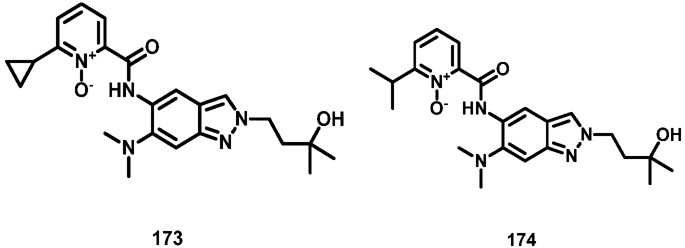

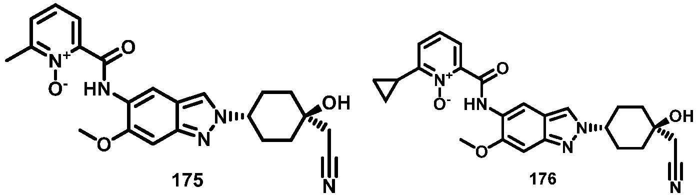

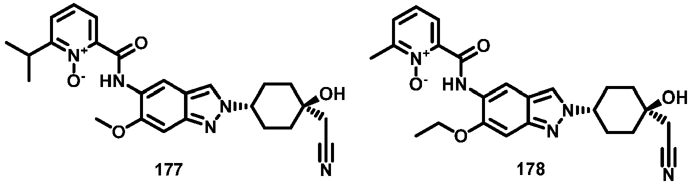

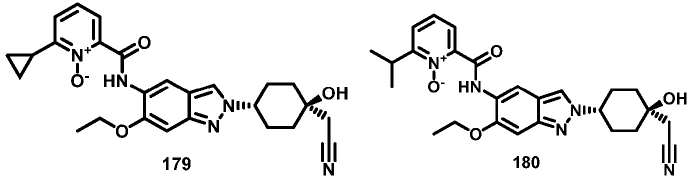

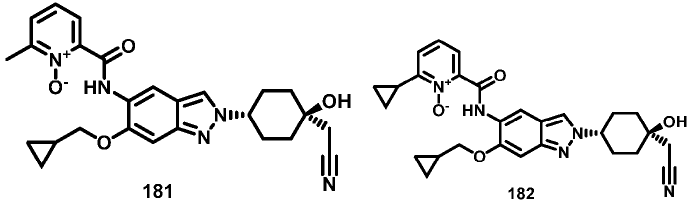

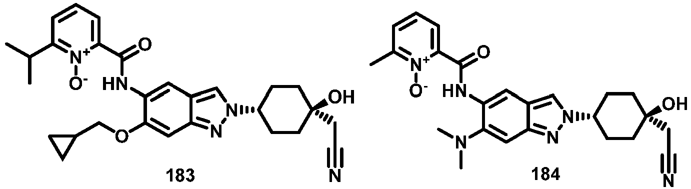

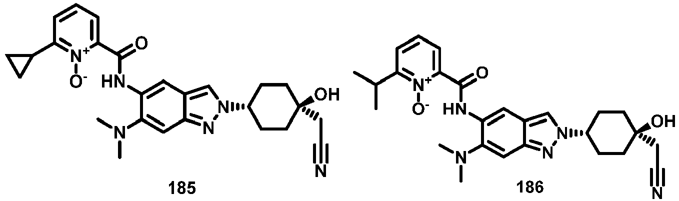

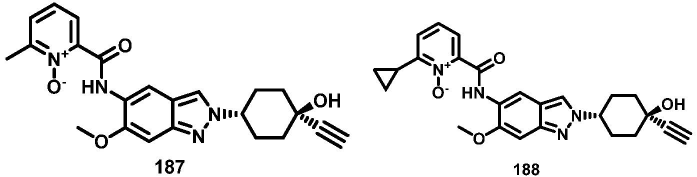

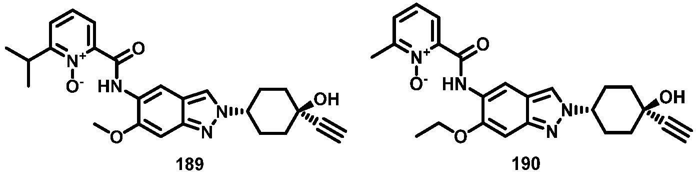

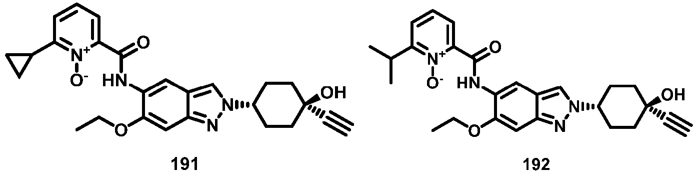

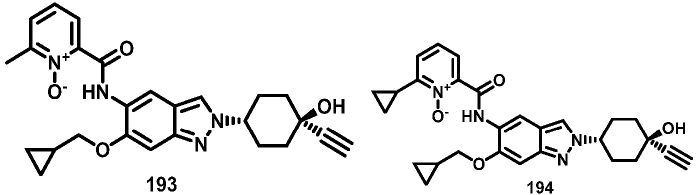

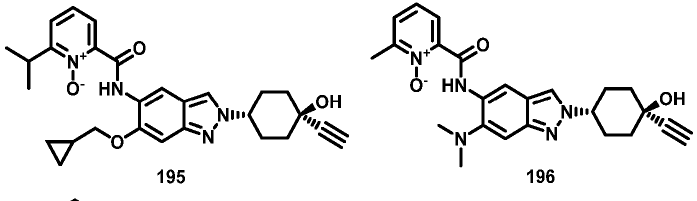

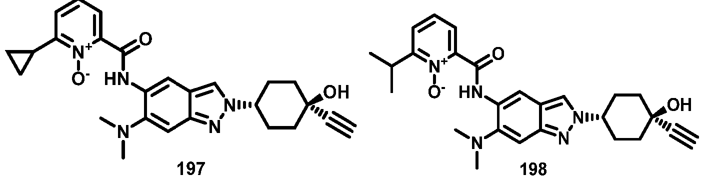

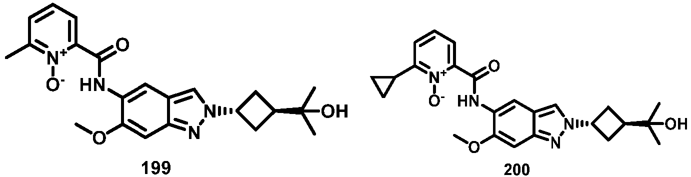

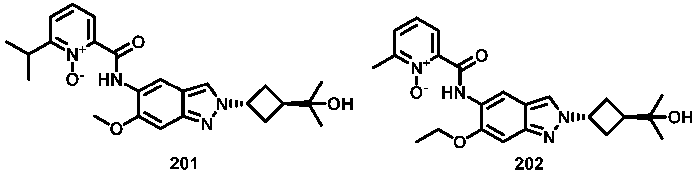

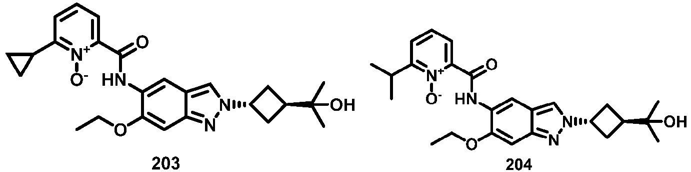

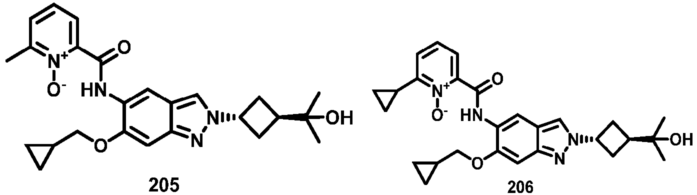

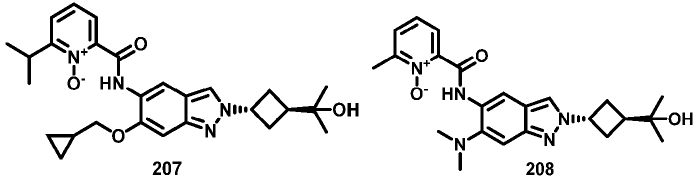

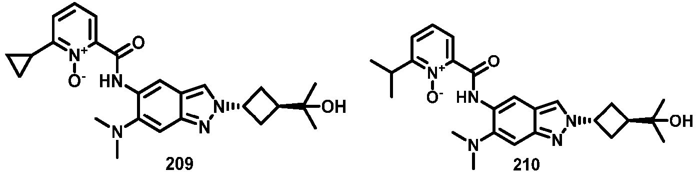

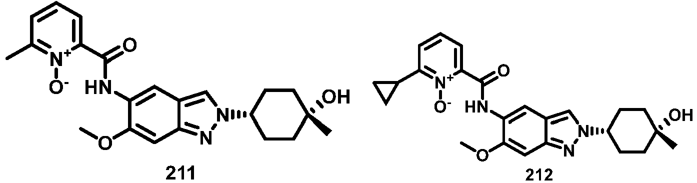

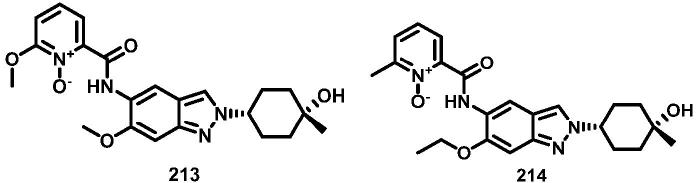

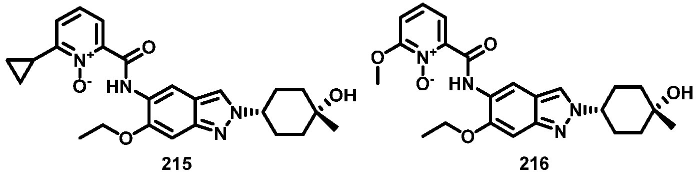

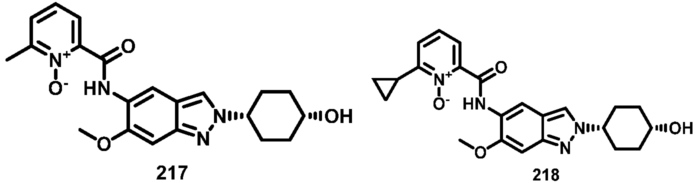

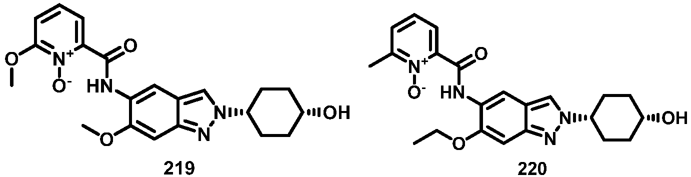

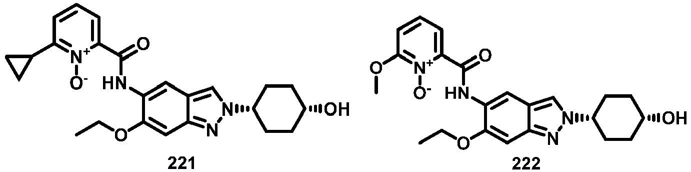

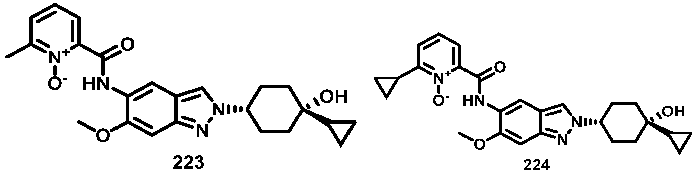

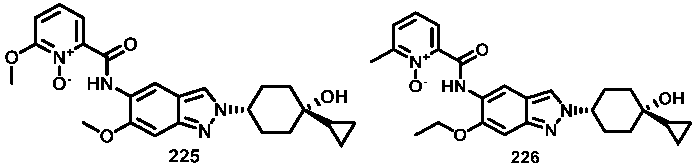

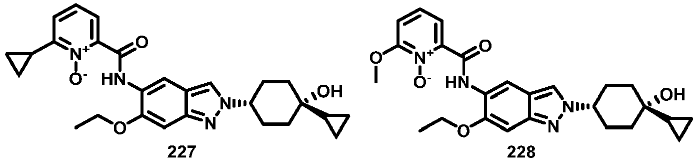

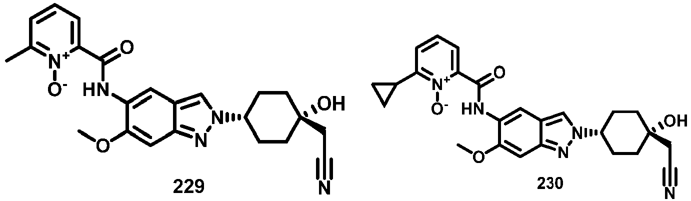

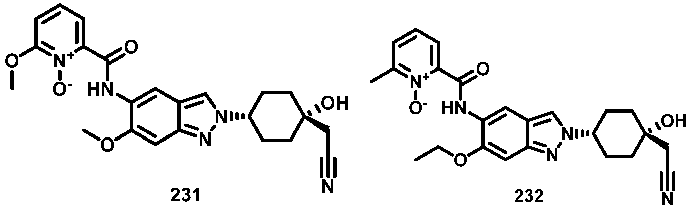

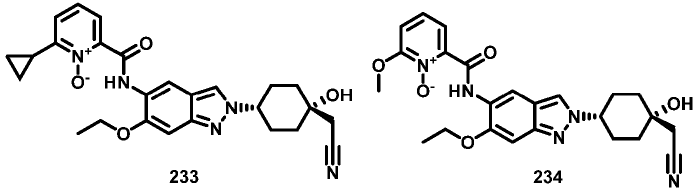

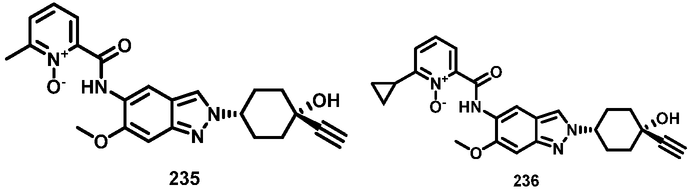

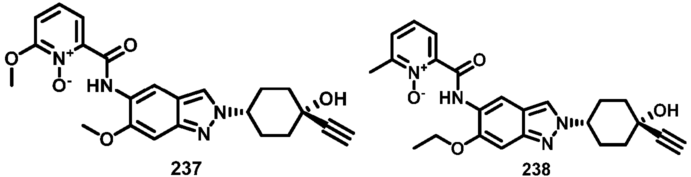

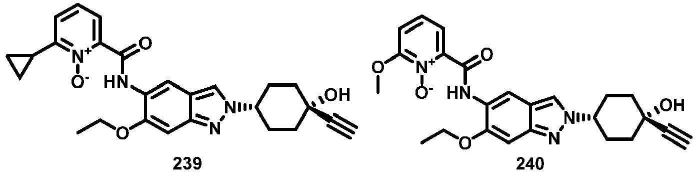

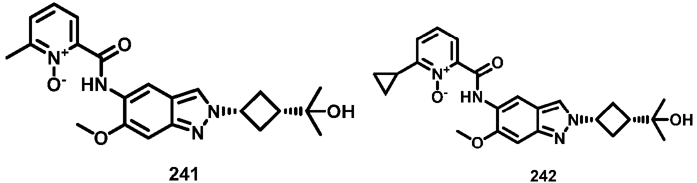

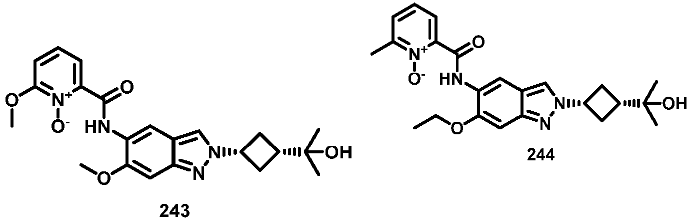

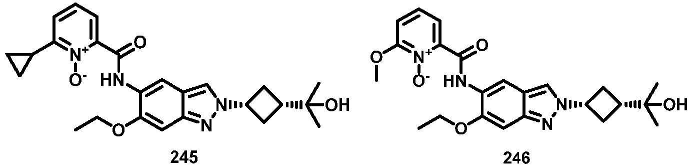

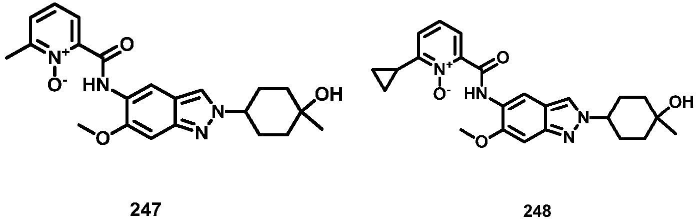

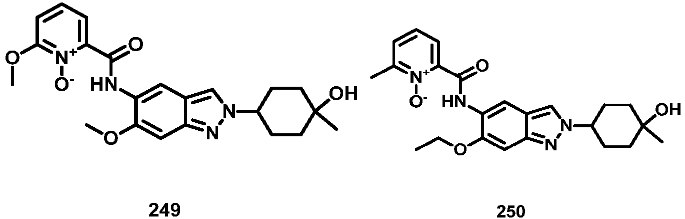

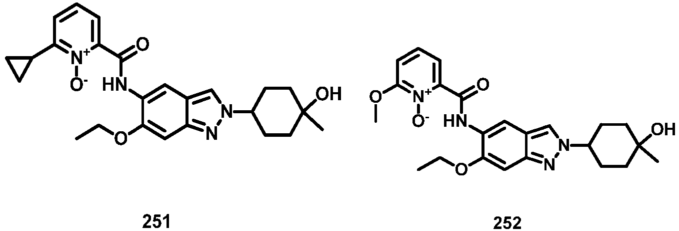

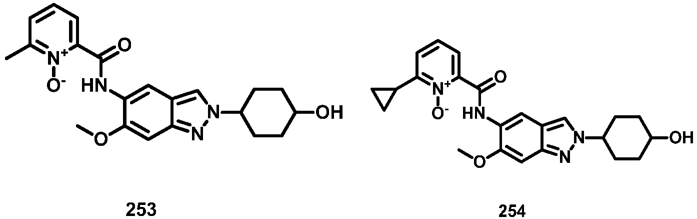

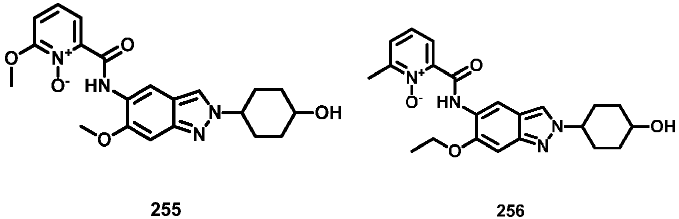

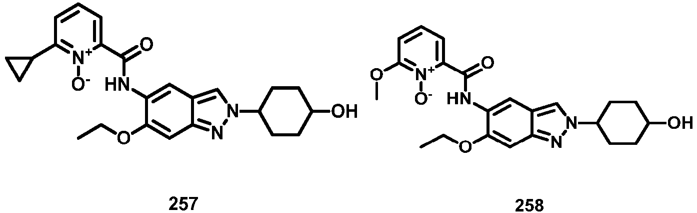

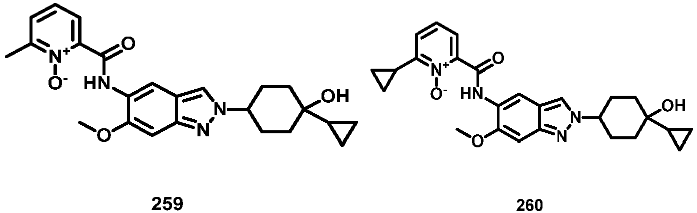

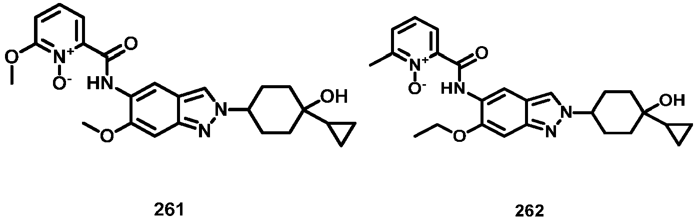

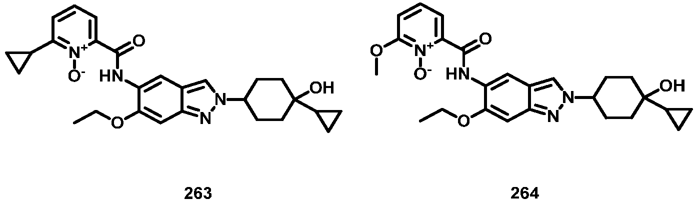

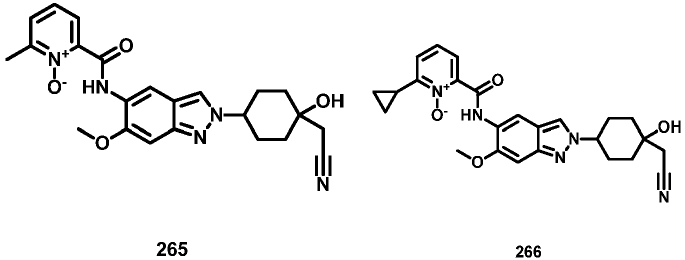

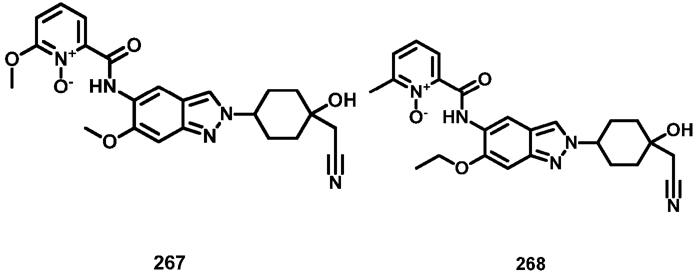

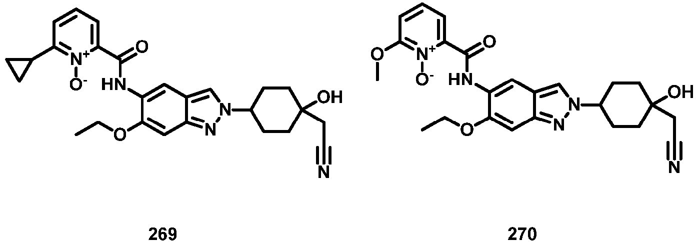

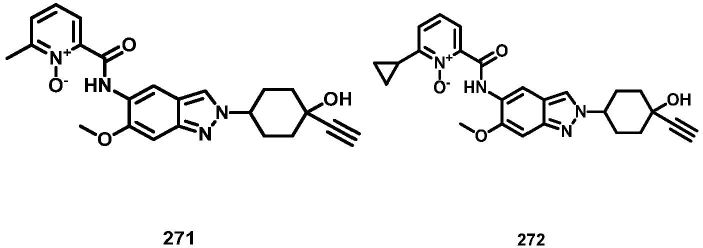

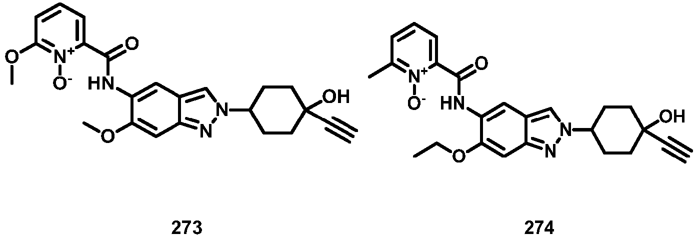

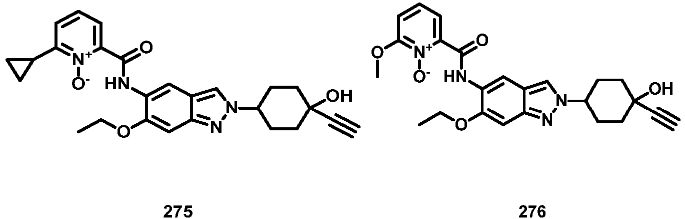

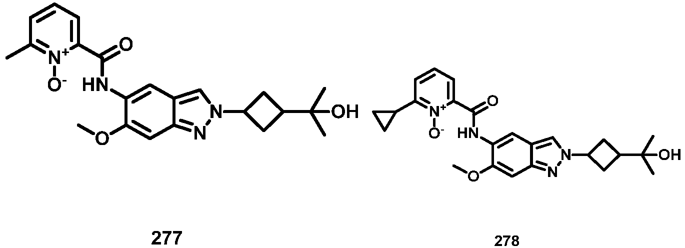

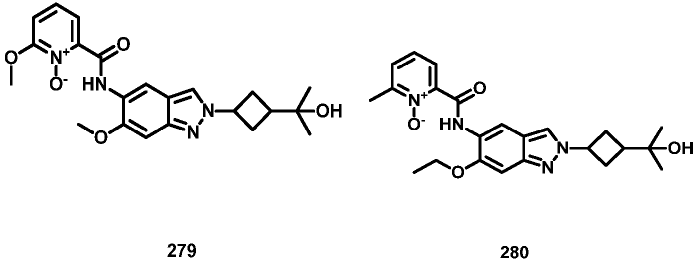

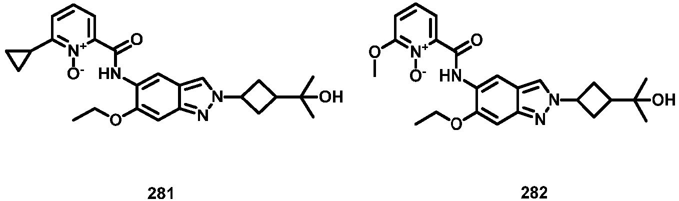

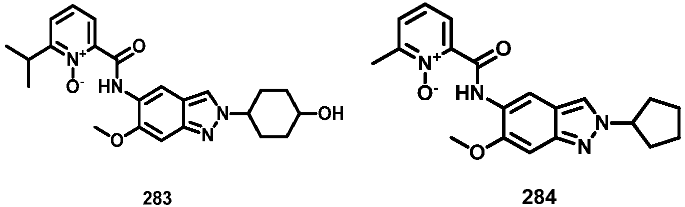

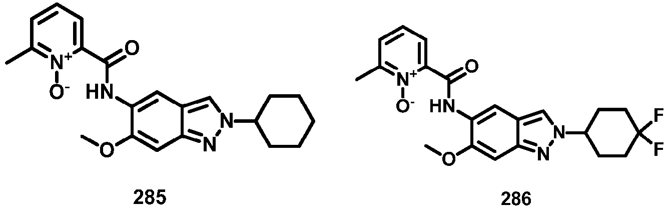

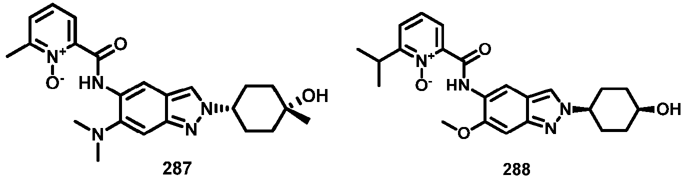

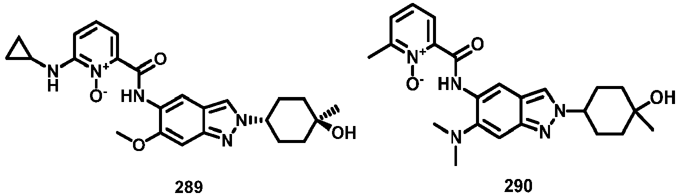

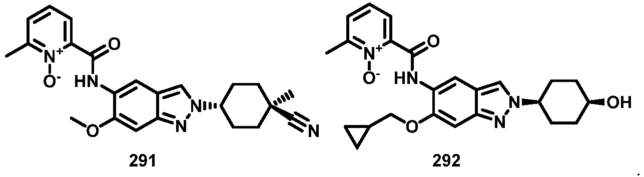

4. The use according to claim 1, wherein the compound of formula I is:

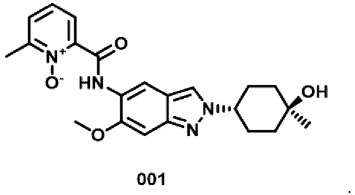

5. The use according to claim 1, wherein the psoriasis is psoriasis vulgaris, psoriatic arthritis, pustular psoriasis, or erythrodermic psoriasis.

6. The use according to claim 1, wherein the compound of formula (I), or a stereoisomer, a racemate, a tautomer, an isotopically labeled compound, a prodrug, or a pharmaceutically acceptable salt thereof, is administered orally, parenterally, or through other routes of administration.

7. The use according to claim 1, wherein the compound of formula (I), or a stereoisomer, a racemate, a tautomer, an isotopically labeled compound, a prodrug, or a pharmaceutically acceptable salt thereof, is preferably administered orally.

8. The use according to claim 7, wherein a dosage form for oral administration is a tablet, film, capsule, sugar-coated tablet, granule, pill, powder, emulsion, suspension, or solution.
